# EUROPEAN PATENT APPLICATION

(11) **EP 3 553 179 A1**
(43) Date of publication of application: **16.10.2019**
(21) Application number: 18167058.9
(22) Date of filing: 12.04.2018
(51) Int. Cl.: C12P 41/00, C12P 5/00, C12P 17/14

(54) **ENANTIOSELECTIVE BIOCATALYTIC PREPARATION OF 4-CYANO-SUBSTITUTED 1-AMINOINDANE AND OZANIMOD**

(71) Applicant: Universität Bielefeld, 33615 Bielefeld (DE); PharmaZell GmbH, 83064 Raubling (DE)
(72) Inventor: Löwe, Jana, 33613 Bielefeld (DE); Uthoff, Florian, 33609 Bielefeld (DE); Harms, Christina, 48231 Warendorf (DE); Gröger, Harald, 33739 Bielefeld (DE); Donsbach, Kai, 83278 Traunstein (DE)
(74) Representative: dompatent von Kreisler Selting Werner - Partnerschaft von Patent- und Rechtsanwälten mbB

(57) **Abstract**

The present invention is directed to a process for the enantioselective biocatalytic preparation of 4-substituted 1-aminoindanes of general formula 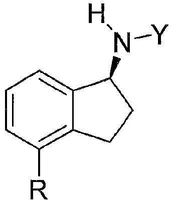 and a process for the preparation of Ozanimod, preferably involving the enantioselective biocatalytic process of preparing 4-substituted 1-aminoindanes of the above general formula, wherein R = -CN and Y = H.

## Description

### Technical Field

The present invention is directed to an enantioselective biocatalytic process for the preparation of 4-substituted 1-aminoindanes as well as a process for the preparation of Ozanimod which process preferably comprises the enantioselective biocatalytic process for the preparation of 4-substituted 1-aminoindanes of the invention.

### Background of the Invention

Ozanimod is an immunomodulatory drug for use in the therapy of relapsing multiple sclerosis (RMS) and ulcerative colitis (UC) having the following structure:

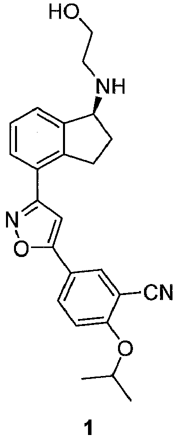

In the synthesis of Ozanimod as disclosed in WO 2015/066515, a chiral primary amine, in particular 4-cyano-substituted 1-aminoindane ((*S*)-2), serves as a key intermediate. This chiral cyano-substituted amine (*S*)-2 of the following structure 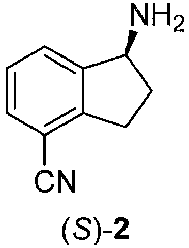 enables access to Ozanimod through functionalization of the amine and nitrile moieties. Chiral amine (*S*)-2 represents an advantageous intermediate since the subsequently required N-alkylation as well as the modification of the cyano-group can be accomplished through well-known and established reaction routes.

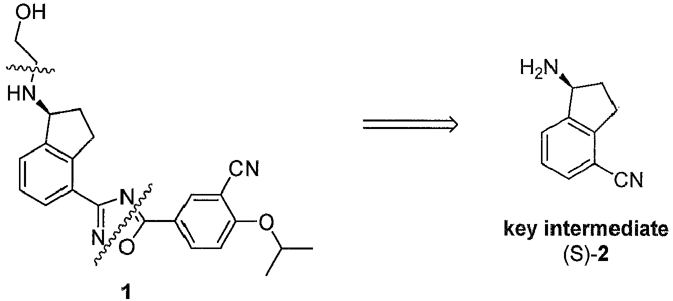

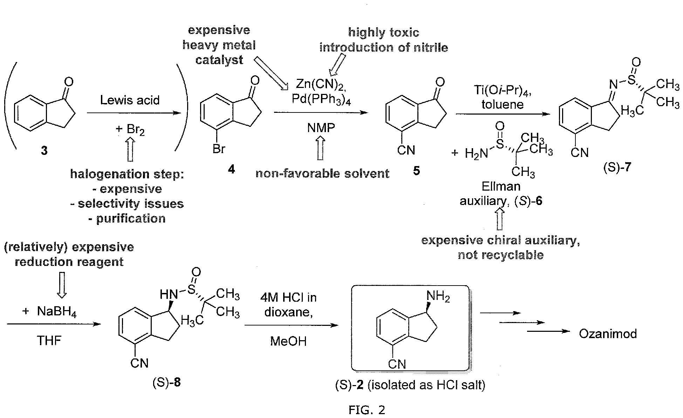

According to the prior art, the synthesis of the amine (*S*)-**2** (obtained as a hydrochloride salt) is carried out by starting from 4-bromo-indanone. An initial palladium-catalyzed cyanation reaction utilizing zinc chloride as a reagent furnishes 4-cyano-1-indanone. The condensation of this ketone with a chiral sulfonamide (*R*)-6 yields the imine and a subsequent diastereoselective reduction followed by cleavage of the chiral sulfonyl group leads to the formation of the desired amine (*S*)-2. This synthetic route disclosed in the prior art is shown in the following Scheme (see also Figure 2):

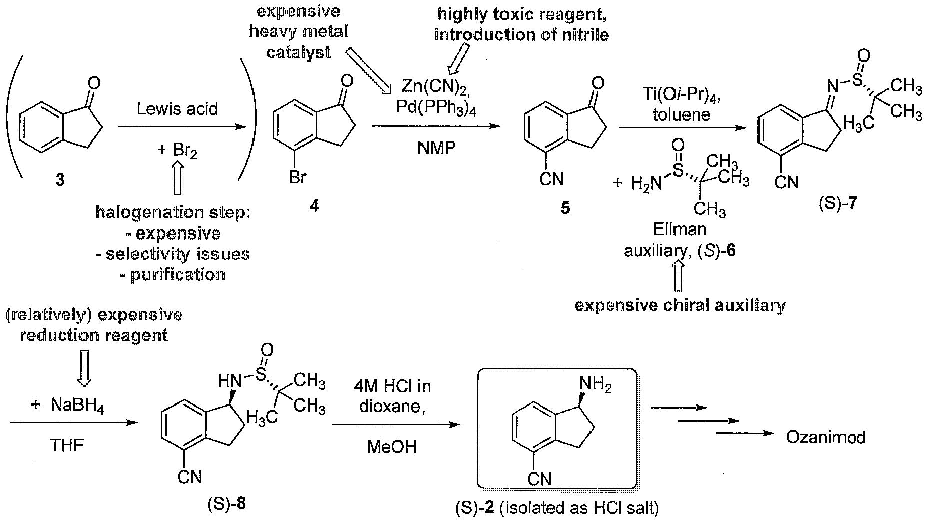

Although this process provides an access to the desired target compound (S)-2, several drawbacks inherent in this multi-step synthetic route have been identified. The initial bromination-step in the synthesis of 4-bromo-indanone (4) is relatively expensive and raises regioselectivity concerns as well as purification issues due to the need for the separation of potential by-products which can be expected. The subsequent cyanation step requires the use of highly toxic cyanide as the reagent in combination with a very expensive heavy metal catalyst (palladium salt). For the formation of the stereogenic center with the desired absolute configuration, a stoichiometric amount of an expensive chiral auxiliary agent, in particular the *Ellman* reagent, is required. In the reduction step, sodium borohydride is used, which represents a relatively expensive hydride source which, in addition, is also less desired in large scale processes. Finally, the chiral sulfonyl group must be cleaved such to result in the formation of the target amine (*S*)-2.

Accordingly, the prior art synthesis according to WO 2015/066515 involves drawbacks with respect to an economical synthesis and some significant specific cost factors. Firstly, the starting material 4-bromo-indanone (4) is a relatively expensive building block. Although 4-bromo-indanone (4) is commercially available, its production is complex and requires quite expensive raw materials. The subsequent cyanation-step is also associated with high costs for reagents and raises safety concerns, e.g., due to the use of highly toxic cyanide. A further drawback is the need of the expensive stoichiometric chiral *Ellman* auxiliary agent which is used in the diastereoselective formation of the stereogenic center of the amine (S)-2.

In light of the drawbacks involved in the route disclosed in WO 2015/066515, it has been an object underlying the present invention to provide a more economically and ecologically attractive as well as technically feasible enantioselective synthesis of chiral (S) 4-substituted 1-aminoindanes in general and, in particular, to (S) 4-cyano-1-aminoindane, i.e. the key intermediate (*S*)-2.

It has been a further object of the present invention to provide an alternative route for the total synthesis of Ozanimod starting from economically attractive compounds, whilst avoiding at least some of the other drawbacks of the prior art.
The solution to these and other objects will become apparent to the person skilled in the art from the present specification, as illustrated in the appended claims.

### Summary of the Invention

Accordingly, the present invention is directed to:
1. A process for the enantioselective biocatalytic preparation of 4-substituted 1-aminoindanes of general formula 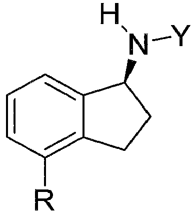 wherein
   R is selected from the group consisting of halogen; -CN; -C(=O)H; -C(=N-OH)H; -C(=O)NH₂; -C(=O)OH; -C(=NH)NH-OH; -C(=N-OH)NH₂; and -COOR¹, wherein R¹ is selected from the group consisting of H; C₁ to C₈-alkyl; arylalkyl, wherein alkyl is C₁ to C₈-alkyl; -CH₂-Hal, wherein Hal is F, Cl or Br; -CH₂-O-alkyl, wherein alkyl is C₁ to C₈-alkyl; optionally substituted aryl; and optionally substituted heteroaryl;
   Y is selected from the group consisting of H; -C(=O)R^{1y}; and - C(=O)OR^{1y}, wherein R^{1y} is selected from the group consisting of H; C₁ to C₈-alkyl; arylalkyl, wherein alkyl is C₁ to C₈-alkyl; -CH₂-Hal, wherein Hal is F, Cl or Br; -CH₂-O-alkyl, wherein alkyl is C₁ to C₈-alkyl; optionally substituted aryl; and optionally substituted heteroaryl;
   via a route selected from the group consisting of
      A resolution of a racemic or enantiomerically-enriched amine mixture of 4-substituted indan-1-amine compounds of general formulae (*S*)-II and (*R*)-II 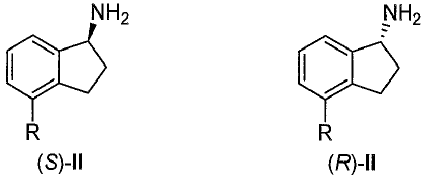
         wherein R is as defined above
         via an enantioselective acylation in the presence of a biocatalyst;
   B resolution of a racemic or enantiomerically-enriched amide mixture of N-acylated 4-substituted indan-1-amine compounds of general formulae (*S*)-III and (*R*)-III 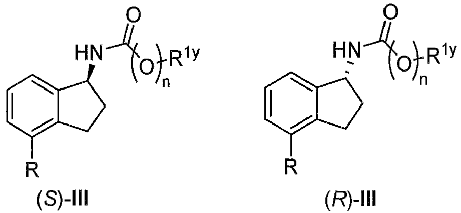
      wherein R and R^{1y} are as defined above and n is 0 or 1,
      via an enantioselective hydrolysis in the presence of a biocatalyst;
   C asymmetric reductive amination of a 4-substituted 1-indanone with a transaminase, wherein the substituent at position 4 is R as defined above; or
   D asymmetric reductive amination of a 4-substituted 1-indanone with an amine dehydrogenase, wherein the substituent at position 4 is R as defined above.
2. The process according to item 1, characterized in that the route is route A or route B, preferably route A.
3. The process according to item 2, wherein the biocatalyst is selected from the group of hydrolases, preferably wherein the hydrolases are selected from the group consisting of lipases, esterases and proteases, most preferably wherein the hydrolase is lipase.
4. The process according to item 2 or 3, characterized in that in route A a racemic amine mixture or an enantiomerically-enriched amine mixture of 4-substituted indan-1-amine compounds of general formulae (*S*)-II and (*R*)-II is added together with an acyl donor to a biocatalyst, preferably wherein the biocatalyst is a lipase.
5. The process according to item 4, characterized in that in route A a racemic amine mixture or an enantiomerically-enriched amine mixture of 4-substituted indan-1-amine compounds of general formulae (*S*)-II and (*R*)-II is added together with an acyl donor to a biocatalyst, preferably wherein the biocatalyst is a lipase, according to the following reaction Scheme 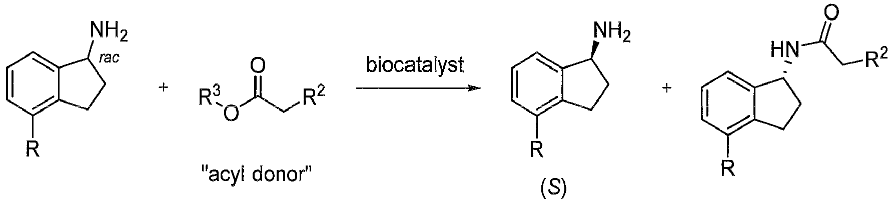 wherein
   R is as defined in item 1, preferably wherein R is -CN, -COOMe, or -Br;
   R² is selected from the group consisting of a C₁ to C₂₀-alkyl group; a C₁ to C₂₀-alkoxy group; an optionally substituted C₆ to C₂₀-aryl group; an optionally substituted 5- or 6-membered heteroaryl group; halogen and an acyl group of formula -COOR⁴, wherein R⁴ is selected from H, and C₁ to C₈-alkyl, preferably -CH₃ or -C₂H₅; preferably wherein R² is methoxy or -COOC₂H₅ or -Cl;
   R³ is selected from a C₁ to C₈- or C₁ to C₆-alkyl group, preferably wherein R³ is a C₁ to C₄-alkyl group, more preferably wherein R³ is methyl, ethyl or isopropyl; -CH₂-Hal wherein Hal represents -F, -Cl or - Br; -CH₂-O-alkyl wherein alkyl represents C₁ to C₈-alkyl; an optionally substituted aryl group; and an optionally substituted 5- or 6-membered heteroaryl group;
   wherein the reaction is conducted in an organic medium in the presence of an organic solvent or under solvent-free conditions.
6. The process according to any one of items 1 to 5, characterized in that R is selected from the group consisting of -Br, -CN and -COOCH₃, preferably wherein R is -CN or -Br, most preferably wherein R is -CN.
7. The process according to any one of items 2 to 6, characterized in that it is carried out in the absence of any additional solvent or in the presence of an additional organic solvent, preferably wherein the organic solvent is at least one selected from the group consisting of n-heptane, 2-methoxy-2-methylpropane, methyl-tert.-butylether, 2-methyl-tetrahydrofuran, methylcyclohexane, toluene and any combination of any thereof, preferably wherein the organic solvent is n-heptane and/or methyl-tert.-butylether.
8. The process according to any one of items 2 to 7, characterized in that the lipase is selected from the group consisting of lipase B from *Candida antarctica* (CAL-B), lipase from *Candida rugosa* (CR lipase), lipase from *Burkholderia cepacia* (BC lipase) and lipase PS from *Burkholderia cepacia,* preferably wherein the lipase is CAL-B.
9. The process according to any one of items 2 to 8, characterized in that the acyl donor is selected from the group consisting of ethyl methoxyacetate, diethyl malonate, and isopropyl methoxyacetate, preferably wherein the acyl donor is isopropyl methoxyacetate.
10. The process according to any one of items 2 to 9, characterized in that the reaction is conducted under one of the following conditions a), b) or d), or wherein the reaction is conducted under a condition selected from conditions a) and b); a) and c); b) and c); and a), b) and c):
   a) for a period of time sufficient to reach 99 % ee of the desired 4-substituted 1-aminoindanes, preferably 30 minutes to 48 hours, more preferably for at least 5 h;
   b) at a temperature range of 10 to 90 °C, preferably at 40 to 60 °C;
   c) at a substrate : enzyme ratio of 1 to 50 mg, preferably 5 mg, CAL-B per mmol substrate;
   preferably in solution in the presence of n-heptane as the solvent.
11. The process according to any one of items 2 to 10, characterized in that the racemic mixture or the enantiomerically-enriched amine mixture of 4-substituted indan-1-amine compounds of general formulae (*S*)-II and (R)-II is obtained from or is obtainable from the corresponding 4-substituted 1-indanone in which the substituent R at the 4-position is as defined in item 1, preferably by
   i) the formation of a ketoxime, particularly by condensation of the 4-substituted 1-indanone with hydroxylamine, followed by
   ii) the reduction of the ketoxime, preferably in the presence of zinc, to the racemic amine mixture.
12. The process according to any one of items 2 to 11, characterized in that the undesired enantiomer(s) resulting from the enzymatic acylation are recovered and recycled/reused.
13. The process according to any one of items 2 to 12, characterized in that a racemic or enantiomerically-enriched amine mixture of 4-substituted indan-1-amine compound of general formulae (*S*)-II and (*R*)-II, wherein R is -CN and Y is hydrogen, is acylated with isopropyl methoxyacetate in the presence of CAL-B in n-heptane and/or methyl-tert.-butylether as the solvent.
14. The process according to any one of items 1 to 13, characterized in that the initial 4-substituted indane-1-one compound is obtainable from or obtained from naphthalene.
15. A process for the preparation of Ozanimod, said process comprising or consisting of the steps, in the given order, of
   I providing naphthalene,
   II reducing naphthalene to 1,2- and/or 1,4-dihydronaphthalene,
      IIa optionally rearranging 1,4-dihydronaphthalene to 1,2-dihydronaphthalene,
   III cleaving the ethylenic C=C-double bond in 1,2-dihydronaphthalene to provide a dicarboxylic acid,
   IV cyclization of the product to 4-carboxy-1-indanone,
   V derivatization of the 4-carboxy-1-indanone to 4-cyano-1-indanone,
   VI converting the 4-cyano-1-indanone to 4-cyano-1-aminoindane in the S-configuration, either directly or via the racemate,
   VII protecting the amino-group of the 4-cyano-1-aminoindane with a protecting group,
   VIII converting the nitrile-group to an amidoxime group,
   IX converting the amidoxime-group to a 1,4-oxadiazole heterocyclic-group via condensation with 3-cyano-4-isopropoxy benzoic acid or 3-cyano-4-isopropoxy benzoic acid derivative,
   X substituting the protecting group on the amino group with a hydroxyethyl group to yield Ozanimod,
   preferably wherein the conversion of the 4-cyano-1-indanone to 4-cyano-1-aminoindane in the S-configuration in step VI is carried out by process according to any one of items 1 or 2 to 14.
16. A process for the preparation of Ozanimod, said process comprising or consisting of the steps, in the given order, of
   VI providing 4-cyano-1-aminoindane in the S-configuration, preferably obtained by the process according to any one of items 1 or 2 to 14,
   VII protecting the amino-group of the 4-cyano-1-aminoindane with a protecting group,
   VIII converting the nitrile-group to an amidoxime group,
   IX converting the amidoxime-group to a 1,4-oxadiazole heterocyclic-group via condensation with 3-cyano-4-isopropoxy benzoic acid or 3-cyano-4-isopropoxy benzoic acid derivative,
   X substituting the protecting group on the amino group with a hydroxyethyl group to yield Ozanimod.

### Brief Description of the Drawings

Figure 1 illustrates the differences between the processes for the preparation of Ozanimod according to the present invention vs. the process outlined in WO 2015/066515.
Figure 2 illustrates some of the disadvantages of the prior art.

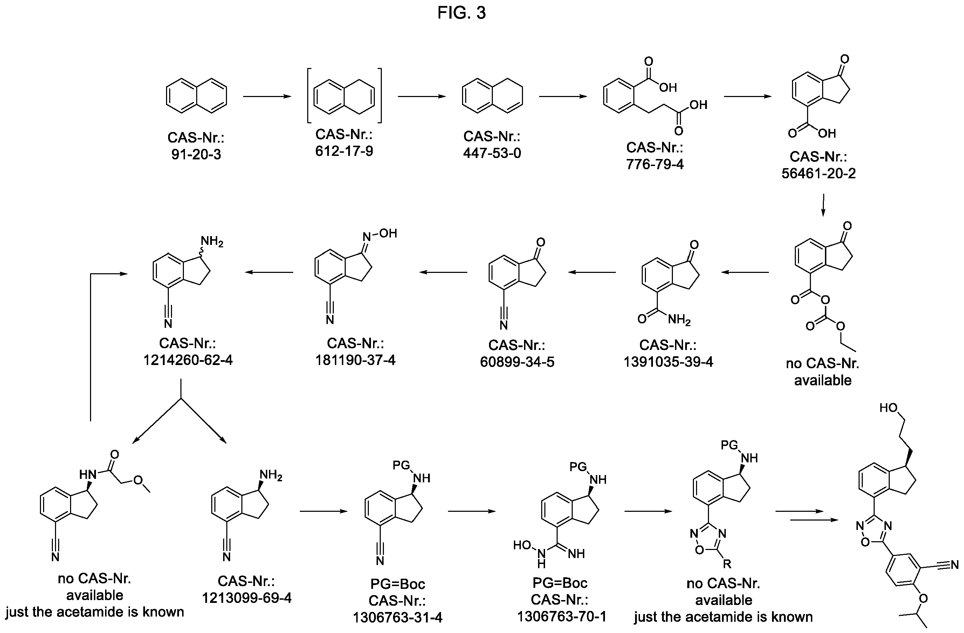

Figure 3 is a non-exhaustive overview of compounds relevant to the present invention (PG = protecting group; Boc = tert.-butyloxycarbonyl group).

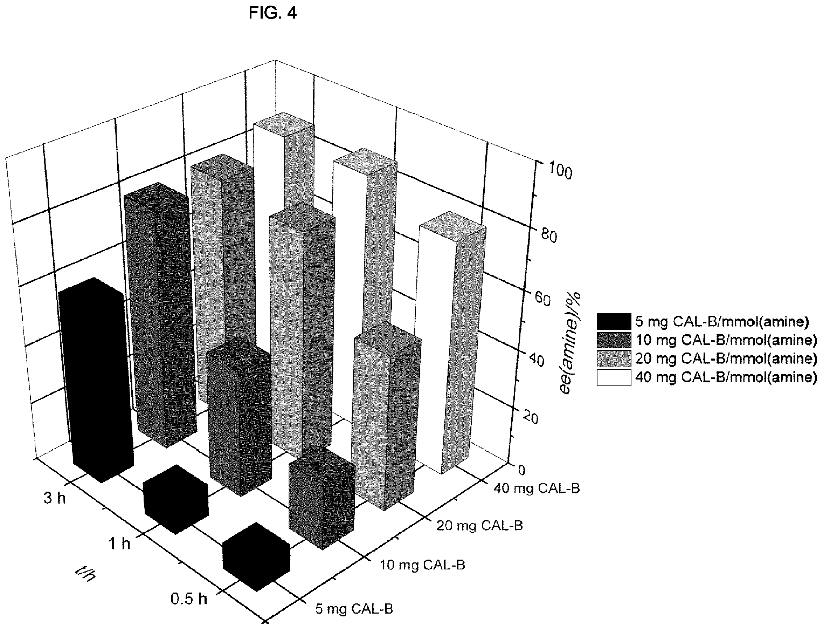

Figure 4 shows the resolution of 1-aminoindane, as an exemplary representation, with lipase B from *Candida antarctica* (CAL-B) at decreased biocatalyst loading conducted at 0.5 M substrate concentration.
Figure 5 shows an acyl donor and solvent screening at 60 °C for the resolution of indan-1-amine with the lipase CAL-B.
Figure 6 shows the resolution of 4-cyano-1-aminoindane (2) at low biocatalyst loading of lipase CAL-B and indicates that a high enantiomeric excess of >95 % ee is reached at a conversion of about 55 %.

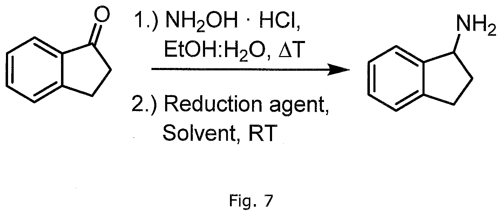

Figure 7 shows the preparation of the racemic 4-substituted indan-1-amines in principle. For illustration only, not according to the invention.

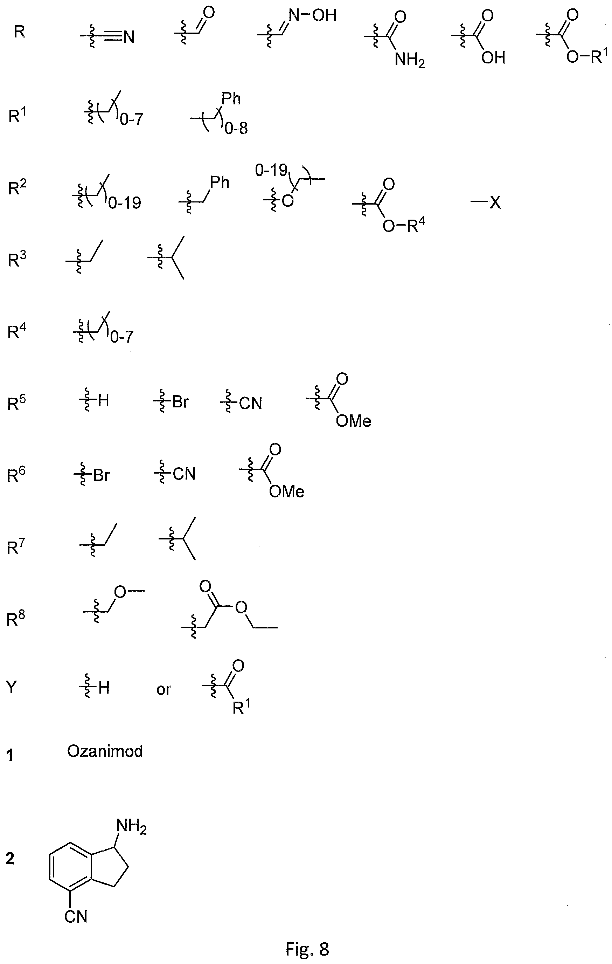

Figure 8 represents an overview of the substituents used in the description of the present invention.

### Terms and Definitions

In context with describing the present invention, the amounts given are amounts by weight, if not stated otherwise.

In the context of the present invention, temperatures are given in degrees Celsius (°C). Reactions, and measurements are conducted at room temperature (23 °C), if not specifically stated otherwise.

In context with the present invention, the process steps are conducted at atmospheric pressure/normal pressure corresponding to about 1013 mbar, if not specifically stated otherwise.

In context with the present invention, the formulation "and/or" is meant to encompass both either one as well as all combinations of the elements listed in the respective lists.

Throughout the present description the term "racemic resolution" means chiral resolution, i.e. in stereochemistry the process for the separation of racemic compounds into their enantiomers.

Enantiomeric excess (ee) is a measurement of purity used for chiral substances. It reflects the degree to which a sample contains one enantiomer in greater amounts than the other. By definition, "enantiomerically pure" means an ee of 100 %; according to the invention it is desired to achieve an ee of the target enantiomer compound of 95 % or above, or 96 % or above, or 97 % or above, or 98 % or above, or 99 % or above.

In the description of the invention, the alkyl-groups may have a straight-chain or may be branched or may by cyclic. In the case of branched-chain and cyclic alkyl-groups, the alkyl-group comprises at least three carbon atoms.

In the description of the invention, the alkoxy-groups may have a straight-chain or may be branched or may by cyclic. In the case of branched-chain and cyclic alkyl-groups, the alkyl-group of the alkoxy-group comprises at least three carbon atoms.

If not specified otherwise hereinbelow, halogen atoms (e.g. Hal) are selected from fluorine (F), chlorine (Cl), bromine (Br) and iodine (I), preferably F, Cl or Br.

Each of the documents referred to herein is incorporated herein by reference, including any prior applications, whether or not specifically listed herein, from which priority is claimed. The mention of any document is not an admission that such document qualifies as prior art or constitutes the general knowledge of the skilled person in any jurisdiction.

Except in the Examples, or where otherwise explicitly indicated, all numerical quantities in this description specifying amounts of materials, reaction conditions, molecular weights, number of carbon atoms, and the like, are to be understood as modified by the word "about". It is to be understood that the upper and lower amount, range, and ratio limits set forth herein may be independently combined. Similarly, the ranges and amounts for each element of the invention can be used together with ranges or amounts for any of the other elements.

As used herein, the transitional term "comprising," which is synonymous with "including," "containing," or "characterized by," is inclusive or open-ended and does not exclude additional, un-recited elements or method steps. However, in each recitation of "comprising" herein, it is intended that the term also encompasses, as alternative embodiments, the phrases "consisting essentially of" and "consisting of", where "consisting of" excludes any element or step not specified and "consisting essentially of" permits the inclusion of additional un-recited elements or steps that do not materially affect the essential or basic and novel characteristics of the composition or method under consideration. Where employed in the present application, the wording "or any combination of any thereof" is to be understood to mean that all possible combinations of the members of the list preceding said wording are directly and unambiguously disclosed in said list. This formulation represents a mere means of formulating the corresponding text (description or claim) in as concise a manner as possible as required under patent law whilst explicitly making clear that said wording is to be read and understood to directly and unambiguously disclose each and every one of the possible combinations arising therefrom.

While certain representative embodiments and details have been shown for the purpose of illustrating the subject invention, it will be apparent to those skilled in this art that various changes and modifications can be made therein without departing from the scope of the subject invention. In this regard, the scope of the invention is to be limited only by the appended claims.

Thus, there has been outlined, rather broadly, the more important features of the invention in order that the detailed description that follows may be better understood and in order that the present contribution to the art may be better appreciated. There are, obviously, additional features of the invention that will be described hereinafter and which will form the subject matter of the claims appended hereto. In this respect, before explaining several embodiments of the invention in detail, it is to be understood that the invention is not limited in its application to the details and construction and to the arrangement of the components set forth in the following description or illustrated in the drawings. The invention is capable of other embodiments and of being practiced and carried out in various ways.

It is also to be understood that the phraseology and terminology herein are for the purposes of description and should not be regarded as limiting in any respect. Those skilled in the art will appreciate the concepts upon which this disclosure is based and that it may readily be utilized as the basis for designating other structures, methods and systems for carrying out the several purposes of this development. It is important that the claims be regarded as including such equivalent constructions insofar as they do not depart from the spirit and scope of the present invention.

### Detailed Description

The present invention relates to a process for the enantioselective biocatalytic preparation of (S) 4-substituted 1-aminoindanes of general formula ((S)-I) 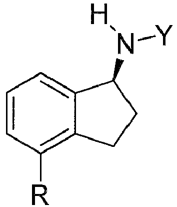 wherein
R is selected from the group consisting of halogen, -CN, -C(=O)H, -C(=N-OH)H, -C(=O)NH₂, -C(=O)OH, -C(=NH)NH-OH, -C(=N-OH)NH₂, and -COOR¹, wherein R¹ is selected from the group consisting of H; C₁ or C₂ to C₈-alkyl, preferably C₁ or C₂ to C₆-alkyl, more preferably C₁ or C₂ to C₄-alkyl; arylalkyl with alkyl representing C₁ or C₂ to C₈-alkyl, preferably C₁ or C₂ to C₆-alkyl, more preferably C₁ or C₂ to C₄-alkyl and wherein aryl is preferably selected from phenyl and naphthyl, preferably phenyl; -CH₂-Hal, wherein Hal is F, Cl or Br; -CH₂-O-alkyl, wherein alkyl is C₁ or C₂ to C₈-alkyl, preferably C₁ or C₂ to C₆-alkyl, more preferably C₁ or C₂ to C₄-alkyl; aryl; and heteroaryl;
Y is selected from the group consisting of H; -C(=O)R^{1y}; and -C(=O)OR^{1y}, wherein R^{1y} is selected from the group consisting of H; C₁ or C₂ to C₈-alkyl, preferably C₁ or C₂ to C₆-alkyl, more preferably C₁ or C₂ to C₄-alkyl; arylalkyl with alkyl representing C₁ or C₂ to C₈-alkyl, preferably C₁ or C₂ to C₆-alkyl, more preferably C₁ or C₂ to C₄-alkyl and wherein aryl is preferably selected from phenyl and naphthyl, preferably phenyl; -CH₂-Hal, wherein Hal is F, Cl or Br; -CH₂-O-alkyl, wherein alkyl is C₁ or C₂ to C₈-alkyl, preferably C₁ or C₂ to C₆-alkyl, more preferably C₁ or C₂ to C₄-alkyl; aryl; and heteroaryl.

In the definition of R¹ and R^{1y}, the aryl group is preferably selected from the group consisting of phenyl, tolyl, xylyl and naphthyl, preferably phenyl, which aryl group may be unsubstituted or substituted with at least one of the substituents selected from the group consisting of H; C₁ or C₂ to C₈-alkyl, preferably C₁ or C₂ to C₆-alkyl, more preferably C₁ or C₂ to C₄-alkyl; arylalkyl with alkyl representing C₁ or C₂ to C₈-alkyl, preferably C₁ or C₂ to C₆-alkyl, more preferably C₁ or C₂ to C₄-alkyl and wherein aryl is preferably selected from phenyl and naphthyl, preferably phenyl; -CH₂-Hal, wherein Hal is F, Cl or Br; -CH₂-O-alkyl, wherein alkyl is C₁ or C₂ to C₈-alkyl, preferably C₁ or C₂ to C₆-alkyl, more preferably C₁ or C₂ to C₄-alkyl, preferably wherein the substituent is selected from C₁ to C₆-alkyl, C₁ to C₄-alkyl and halogen.

In the definition of R¹ and R^{1y}, the heteroaryl group is preferably a 5- or 6-membered heterocyclic group containing at least one heteroatom selected from the group consisting of nitrogen, oxygen and sulfur, preferably a species selected from the group consisting of pyridyl, pyrimidyl, furyl, thienyl, and imidazolyl; which heteroaryl group may be unsubstituted or represent a substituted heteroaryl group selected from pyridyl, pyrimidyl, furyl, thienyl, and imidazolyl, wherein the substituent is selected from C₁ to C₆-alkyl, C₁ to C₄-alkyl and halogen.

Preferably R^{1y} is C₁ to C₄-alkyl (more preferably selected from methyl, ethyl, n- or iso-propyl, and tert.-butyl, most preferably tert.-butyl) or arylalkyl wherein the alkyl-group is C₁ or C₂-alkyl and preferably wherein aryl the aryl-group is phenyl.

In one embodiment R is selected from halogen, preferably -Br; -CN and -COOR¹, such as -COOCH₃, most preferably -CN; and Y is H or -C(=O)R^{1y}, preferably wherein R^{1y} is a C₁ to C₄-alkyl group, more preferably selected from a methyl, ethyl group and tert.-butyl group, most preferably wherein R^{1y} is a tert.-butyl group.

In one embodiment of the present invention, R is selected from -Br, -CN or -COOCH₃, preferably wherein R is -CN and Y is H or -C(=O)R^{1y}, wherein R^{1y} is the tert.-butyl group.

In one embodiment R is -CN and Y is H, corresponding to the most preferred target amine enantiomer in S-configuration, i.e. 4-cyano-1-aminoindane (S)-2.

For the purpose of enantioselectively preparing the 4-substituted 1-aminoindanes of general formula I of the invention, such as the target amine enantiomer (*S*)-2, four alternative pathways/routes are particularly encompassed by the present invention:
- When transforming a 4-substituted 1-indanone as described above into the corresponding racemic amine, a lipase-catalyzed resolution is used as a biocatalytic option as route A - representing the preferred route according to the present invention.
- Route B relates to the resolution of a racemic mixture or an enantiomerically-enriched mixture of a suitable amide via an enantioselective hydrolysis.
- As routes C and D, the direct asymmetric reductive amination of a 4-substituted 1-indanone as described above is provided, involving either transaminases (route C) or amine dehydrogenases (route D) as the catalysts.

Route A relates to the resolution of a racemic amine via an N-acylation in the presence of a biocatalyst as key step, preferably in the presence of a hydrolase, preferably selected from a lipase, esterase or protease. Most preferably a lipase is used as a biocatalyst. The formation of the enantiomerically pure 4-cyano-substituted 1-aminoindane (i.e. compound (*S*)-2)) in an early stage of the total synthesis of Ozanimod according to WO 2015/066515 renders the use of a resolution process for the generation of the enantiomerically pure 4-cyano-substituted 1-aminoindane particularly attractive. Accordingly, one aspect of the present invention is directed to the resolution of a racemic amine mixture of 4-substituted indan-1-amines of general formulae *(S)*-II and *(R)*-II as described above via their acylation in the presence of a biocatalyst, preferably in the presence of a lipase as a key step. Instead of a racemic mixture, an enantiomerically-enriched mixture of a suitable amine can similarly be used as a substrate for this process.

Route B relates to the resolution of a racemic amide via hydrolysis in the presence of a biocatalyst as key step, preferably in the presence of a hydrolase, preferably selected from a lipase, esterase or protease. Most preferably a lipase is used as a biocatalyst. One aspect of the present invention is directed to the resolution of a racemic amide mixture of 4-substituted indan-1-amides of general formulae (*S*)-III and (*R*)-III as described above via enantioselective hydrolysis with a biocatalyst as key step. Instead of a racemic mixture also an enantiomerically-enriched mixture of a suitable amide can be used as a substrate for this process.

Route C towards the 4-substituted 1-aminoindane target compounds (such as 4-cyano-1-aminoindane, (*S*)-2 or related indan-1-amines with other substituents in 4-position) consists of the direct asymmetric reductive amination of the corresponding indanone substrates. Such reductive amination can be carried out using transaminases in combination with an amine donor, which, in said conversion, is oxidized to form a by-product.
In this route an enantioselective transaminase, e.g. transaminase from *Vibrio fluvialis* (VF-TA) or transaminase from *Arthrobacter sp.* (ArS-TA), is employed as biocatalyst in combination, particularly with a co-factor, e.g. pyridoxal phosphate, and a suitable amine donor. As such an amine donor particularly either L-alanine or isopropylamine can be used, leading to the oxidation products pyruvate or acetone. This reaction can be carried out in either aqueous or organic media.
Particularly, this route can be carried out with VF-TA. An exemplary process is conducted with L-alanine in aqueous medium, methanol 20 % v/v, Pᵢ-buffer (0.1 M, pH 8.0) at 30 °C for 24 hours.

In route D the asymmetric reductive amination of ketone is carried out utilizing an amine dehydrogenase (AmDH) with NAD(P)H as co-factor instead of a transaminase as a biocatalyst. In this route the required nitrogen component (nitrogen source) can be ammonia. A reducing agent is required which can be, for example, D-glucose or formate. In addition, a second enzyme for *in situ*-cofactor-regeneration is needed which can a glucose dehydrogenase (in case of D-glucose as a co-substrate) or a formate dehydrogenase (in case of formate as a co-substrate).
One example of a usable amine dehydrogenase is the artificial amine dehydrogenase reported by Zheng and Xu et al. (ChemCatChem 2015, 7, 3838-3841), who mutated a leucine dehydrogenase from *Exigobacterium sibiricum* (EsLeuDH-DM) in two positions (K77S/N270L).

In one embodiment, the most preferred alternative route for the preparation of the desired 4-substituted 1-aminoindanes (such as compound (*S*)-2 and related analogues with other 4-substituents) is represented by the enzymatic resolution of racemic underlying amines through enantioselective acylation in the presence of a lipase is are routes A and B, the most preferred embodiment is route A.

In the embodiment of route A and B, the present invention is directed to an enantioselective biocatalytic process for the preparation of the 4-substituted 1-aminoindanes of general formula I as defined above,
(i) via route A of resolving a racemic mixture of (chiral) 4-substituted indane-1-amine enantiomers of general formula II 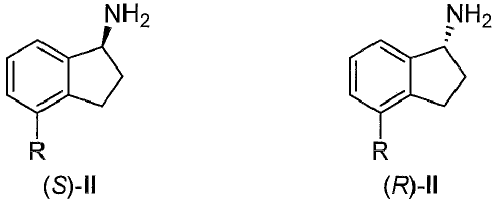
   wherein R is as defined above, preferably halogen (-Cl or -Br), -COOCH₃, or -CN, most preferably -CN;
   via an enantioselective acylation (with an acylating agent described herein) in the presence of a hydrolase, preferably wherein the hydrolase is selected from the group consisting of a lipase, esterase and protease, most preferably in the presence of a lipase, or
(ii) via route B of resolving a racemic amide mixture of (chiral) 4-substituted indane-1-amides enantiomers of general formulae *(S)*-I and *(R)*-I 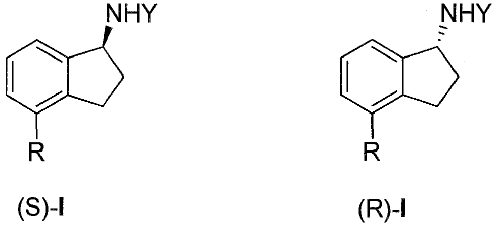 wherein R is as defined above, preferably halogen (-CI or -Br), -COOCH₃, or -CN, most preferably -CN; and Y is as defined above, preferably wherein Y is -C(=O)R^{1y} or -C(=O)OR^{1y}, wherein R^{1y} is as defined above, most preferably wherein R^{1y} is C₁ to C₄-alkyl (in one preferred embodiment selected from methyl, ethyl, n-propyl, isopropyl and tert.-butyl, most preferably tert.-butyl) or arylalkyl, wherein alkyl is C₁ or C₂-alkyl and aryl representing phenyl.
*via* enantioselective hydrolysis in the presence of a biocatalyst, preferably in the presence of a hydrolase, most preferably wherein the hydrolase is selected from the group consisting of a lipase, esterase and protease, most preferably in the presence of a lipase.

A preferred embodiment of invention route B is directed to the resolution of a racemic or enantiomerically-enriched (amide) mixture of N-acylated 4-substituted indan-1-amine compounds of general formulae (*S*)-III and (*R*)-III 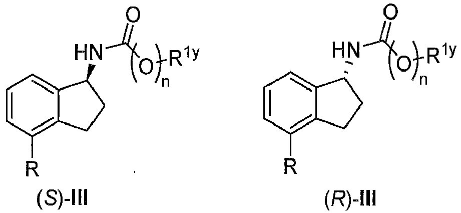
wherein R and R^{1y} are as defined above and n is 0 or 1, preferably wherein n is 0,
via enantioselective hydrolysis in the presence of a biocatalyst.

In a preferred further embodiment R^{1y} is C₁ to C₄-alkyl (such as methyl, ethyl, propyl, isopropyl or tert.-butyl) or arylalkyl with alkyl representing C₁ or C₂-alkyl and aryl representing phenyl. In a still further embodiment R is -CN.

In a still further preferred embodiment R represents -CN, n is 0, and R^{1y} is C₁ to C₄-alkyl, more preferably R^{1y} is selected from the group consisting of a methyl, ethyl and a tert.-butyl group, most preferably R^{1y} is a tert.-butyl group.

In one embodiment of the present invention, the process is characterized in that in route A the racemic amine mixture or an enantiomerically-enriched amine mixture of 4-substituted indan-1-amine compounds of general formulae (*S*)-II and (*R*)-II is added to a biocatalyst, preferably to a lipase together with an acyl donor (i.e. an acylating agent) according to the following general exemplary reaction Scheme (exemplifying biocatalyst = lipase; reaction medium = organic solvent): 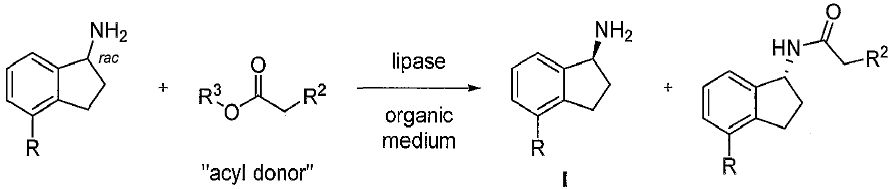 wherein
R is as defined above, preferably halogen (-CI or -Br), -COOCH₃, or -CN, most preferably -CN;
R² is C₁ to C₂₀-alkyl, C₁ or C₂ to C₁₈-alkyl, or C₁ or C₂ to C₁₆-alkyl, or C₁ or C₂ to C₁₂-alkyl, or C₁ or C₂ to C₈-alkyl, or C₁ or C₂ to C₆-alkyl, or C₁ or C₂ to C₄-alkyl; C₁ to C₂₀-alkoxy, C₁ or C₂ to C₁₈-alkoxy, or C₁ or C₂ to C₁₆-alkoxy, or C₁ or C₂ to C₁₂-alkoxy, or C₁ or C₂ to C₈-alkoxy, or C₁ or C₂ to C₆-alkoxy, or C₁ or C₂ to C₄-alkoxy; an optionally substituted aryl group having 6 to 20 carbon atoms, preferably wherein the aryl group is as defined above in conjunction with the definition of R¹/R^{1y}; an optionally substituted 5- or 6-membered heteroaryl group as defined above in conjunction with the definition of R¹/R^{1y}, or an acyl group of formula -COOR⁴, wherein R⁴ is an C₁ or C₂ to C₈-alkyl, preferably C₁ or C₂ to C₆-alkyl, more preferably C₁ or C₂ to C₄-alkyl, particularly CH₃ or C₂H₅; a halogen atom; preferably wherein R² is selected from the group consisting of methoxy, -COOC₂H₅ and chlorine;
R³ is selected from the group consisting of C₁ or C₂ to C₈-alkyl, preferably C₁ or C₂ to C₆-alkyl, more preferably C₁ or C₂ to C₄-alkyl, preferably R³ is selected from the group consisting of methyl, ethyl and isopropyl, most preferably wherein R³ is ethyl; -CH₂-Hal with Hal representing -F, -Cl or -Br; -CH₂-O-alkyl with alkyl representing C₁ to C₈-alkyl; an optionally substituted aryl group as defined above in conjunction with the definition of R¹/R^{1y}; or an optionally substituted 5- or 6-membered heteroaryl group as defined above in conjunction with the definition of R¹/R^{1y};
wherein the reaction is conducted in an organic medium either with an additional organic solvent or under neat conditions (i.e. solvent-free).
In one embodiment preferred acylating agents are characterized by R³ being selected from a C₁ to C₄-alkyl group (such as a methyl, ethyl, propyl, isopropyl group, n- and tert.-butyl group, preferably ethyl and isopropyl group) and R² being selected from the group consisting of methoxy, -COOC₂H₅ and chlorine, preferably methoxy.

The reaction principle is hereinafter shown for a selected racemic amine in the following reaction Scheme (for example, wherein R = -CN and R² = methoxy):

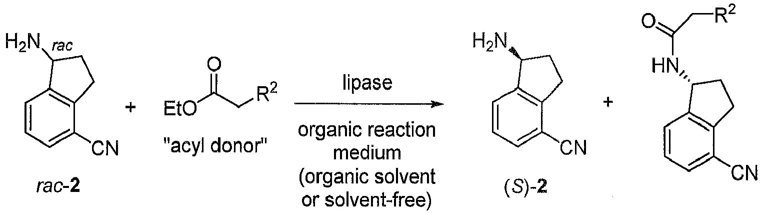

With respect to the enzymatic resolution of Ozanimod-related racemic amines and taking into account the maximum yield of 50 %, such resolution method is attractive, in particular when applying it at a relatively early stage of the multi-step synthesis. At such relatively early stage in the resolution, the 4-substituted indan-1-amine *rac*-2 (or the other 4-substituted analogues) can be considered substrates. Besides high process efficiency, the recovery of the undesired enantiomer and racemization is similarly desirable for different purposes.

In one embodiment of the present invention, the organic solvent is selected from the group consisting of n-heptane, 2-methoxy-2-methylpropane, methyl-*tert*.-butylether (MTBE), 2-methyltetrahydrofuran (2-MTHF), methylcyclohexane (MCH), toluene and mixtures thereof, preferably wherein the solvent is selected from n-heptane, MTBE and toluene, or mixtures thereof.

In one embodiment of the present invention, the lipase is selected from the group consisting of lipase B from *Candida antarctica* (CAL-B), lipase from *Candida rugosa* (CR lipase), and lipase from *Burkholderia cepacia* (BC lipase), preferably CAL-B. The lipase B from *Candida antarctica* (CAL-B) is one of two different lipases (lipase A and lipase B) produced by said organism.
CAL-B is commercially available as a recombinant, immobilized enzyme (e.g. Novozyme 435). The lipase from *Candida rugosa* is isolated from fungus, and the lipase from *Burkholderia cepacia* is isolated from bacteria. The lipases mentioned above are commercially available. According to an embodiment of the invention, the use of lipase B from *Candida antarctica* (CAL-B) is particularly preferred.
The resolutions using the lipase catalysts are typically carried out in an organic medium, preferably wherein the biocatalyst is in immobilized form. This enables a simple separation of the product mixture from the enzyme component. This kind of enzymatic resolution involving a suitable acylation agent offers a wide range of advantages.

In general, according to the invention, any type of acyl donor, i.e. acylating agent can be used. In one embodiment of the present invention, the acyl donors are esters, in particular those having structures such as those shown above in Scheme I. Representative esters are selected from the group consisting of alkyl 2-butanoates, alkyl octanoates, dialkyl oxalates, dialkyl malonates, alkyl 2-chloroacetate, wherein the alkyl group is selected from C₁ to C₆, preferably C₁ to C₄-alkyl groups, wherein alkyl groups such as methyl, ethyl or isopropyl, are preferred.

In one embodiment of the present invention, the acyl donor is selected from the group consisting of ethyl methoxyacetate, diethyl malonate, and isopropyl methoxyacetate, preferably wherein the acyl donor is isopropyl methoxyacetate.

In embodiments of the present invention, the reaction is conducted under one of the following, two of the following (i.e. (a) and b), a) and c), b) and c)) or all three of the following conditions:
a) for a time sufficient to reach 99 % ee or more of the desired 4-substituted 1-aminoindanes (as remaining substrates), preferably for 30 minutes to 48 hours, more preferably at least 5 h such to reach 99 % ee or more of the desired 4-substituted 1-aminoindanes;
b) in a temperature range of 10 to 90 °C, preferably 40 to 60 °C;
c) with a substrate : enzyme ratio of 1 to 50 mg CAL-B per mmol substrate,
preferably in the presence of an organic solvent selected from those specified above, more preferably in the presence of n-heptane.
It is apparent that the time sufficient to reach at least 99 % ee of the desired 4-substituted 1-aminoindanes in condition a) depends on the parameters chosen for conditions b) and c) and can easily be controlled according to the respective reaction conditions chosen.

In one embodiment of the present invention, the racemic mixture, i.e. the substrate used in the subsequent enantioselective, biocatalytic resolution method (route A), is prepared from the corresponding 4-substituted 1-indanone in which the substituent R at the 4-position is defined as above. In one embodiment this can be accomplished by
i) the formation of a ketoxime, particularly by condensation of the 4-substituted 1-indanone with hydroxylamine, followed by
ii) the reduction of the ketoxime, particularly in the presence of zinc, resulting in the racemic amine mixture.

In one embodiment of the present invention, the undesired enantiomer(s) resulting from the enzymatic acylation are recovered and recycled/reused.

In one preferred embodiment of the present invention, the process for making the enantiomer of formula *(S)*-I is characterized in that a racemic or enantiomerically-enriched amine mixture of 4-substituted indan-1-amine compound of general formulae (*S*)-II and (*R*)-II, wherein R is -CN and Y is hydrogen, is acylated with isopropyl methoxyacetate in the presence of CAL-B in n-heptane and/or MTBE, preferably in the presence of n-heptane as the solvent.

In one further embodiment of the present invention, the initial starting compound from which the 4-substituted indane-1-one is prepared, is naphthalene.

In a still further embodiment, the present invention is directed to a process for the preparation of Ozanimod comprising or consisting of the steps, in the given order, of
I providing naphthalene,
II reducing naphthalene to 1,2- and/or 1,4-dihydronaphthalene,
   IIa optionally rearranging 1,4-dihydronaphthalene to 1,2-dihydronaphthalene,
III cleaving the ethylenic C=C-double bond in 1,4-dihydronaphthalene to yield a dicarboxylic acid,
IV cyclization of the product to form 4-carboxy-1-indanone,
V derivatizing the 4-carboxy-1-indanone to form 4-cyano-1-indanone,
VI converting the 4-cyano-1-indanone to form 4-cyano-1-aminoindane in the *S*-configuration, either directly or via the racemate;
VII protecting the amino-group of the 4-cyano-1-aminoindane (2) with a protecting group,
VIII converting the nitrile-group to form an amidoxime group,
IX converting the amidoxime-group to form an 1,4-oxadiazole heterocyclic-group via condensation with 3-cyano-4-isopropoxy benzoic acid or 3-cyano-4-isopropoxy benzoic acid derivative, and
X substituting the protecting group on the amino group with a hydroxyethyl group to yield Ozanimod.

In one embodiment of the present invention, the conversion of the 4-cyano-1-indanone to 4-cyano-1-aminoindane in the (S)-configuration in step VI above is achieved through a process according to any one of the embodiments according to the invention outlined further above, preferably through route A (involving the racemate). Accordingly, in one embodiment, the present invention also relates to a method for the preparation of Ozanimod comprising the step of providing 4-cyano-1-aminoindane in the *S*-configuration according to the method of route A or route B (after separating the desired S-enantiomer and deacylation). Preferably, the synthesis of Ozanimod comprises the method of route A.

The present invention, in a yet more specific embodiment, is further directed to a process for the preparation of Ozanimod comprising or consisting of the steps, in the given order, of
VI providing 4-cyano-1-aminoindane in the *S*-configuration, preferably obtained by or obtainable by a process according to any one of the embodiments outlined further above, more preferably according to route A or route B (after separating the desired S-enantiomer and deacylation),
VII protecting the amino-group of the 4-cyano-1-aminoindane with a protecting group,
VIII converting the nitrile-group to an amidoxime group,
IX converting the amidoxime-group to a 1,4-oxadiazole heterocyclic-group via condensation with 3-cyano-4-isopropoxy benzoic acid or 3-cyano-4-isopropoxy benzoic acid derivative, and
X substituting the protecting group on the amino group with a hydroxyethyl group to yield Ozanimod.

In one embodiment of the present invention in step II, the reduction of naphthalene to 1,2-dihydronaphthalene is conducted either electrochemically or *via* the *Birch* reaction. In one particular embodiment the *Birch* reaction is conducted in the presence of sodium and *tert*.-butanol in diethyl ether (see 1 a) T. Asahara, M. Seno, H. Kaneko, Bulletin of the Chemical Society of Japan 1968, 41, 2985; b) A. Misono, T. Osa, T. Yamagishi, Bulletin of the Chemical Society of Japan 1967, 40, 427; c) J. Mortensen, J. Heinze, Angew. Chem. Int. Ed. Engl. 1984, 23, 84; d) H. W. Sternberg, R. Markby, I. Wender, Journal of The Electrochemical Society 1963, 110, 425 for electrochemical synthesis; 2 a) O. S. Kukovinets, M. I. Kislitsyn, R. A. Zainullin, A. A. Mukhamedzyanova, F. Z. Galin, M. I. Abdullin, Russian Journal of Organic Chemistry 2008, 44, 362; b) A. Menzek, A. Altundas, D. Gültekin, Journal of Chemical Research 2003, 2003, 752 for Birch type reaction).

In one embodiment of the present invention, an additional step IIa can be carried out, i.e. the rearrangement of 1,4-dihydronaphthalene to 1,2-dihydronaphthalene, if the ratio of 1,4-dihydronaphthalene to 1,2-dihydronaphthalene resulting from step II is not favorable. Step IIa, in one particular embodiment, is conducted either in the presence of a strong acid or in the presence of a strong base, such as, in particular, potassium *tert.-*butoxide in tert.-butanol, more particularly at a temperature of about 60 °C for about 12 hours (see a) S. Suzuki, M. Kato, S. Nakajima, Can. J. Chem. 1994, 72, 357; b) W. Wu, J. G. Verkade, ARKIVOC 2004, 88; c) D. R. Vutukuri, P. Bharathi, Z. Yu, K. Rajasekaran, M.-H. Tran, S. Thayumanavan, J. Org. Chem. 2003, 68, 1146; d) M. Güney, A. Co 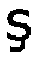 kun, F. Topal, A. Da 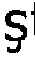 tan, I. Gülçin, C. T. Supuran, *Bioorganic & Medicinal Chemistry* 2014, 22, 3537 for dihydronaphthalene rearrangement).

In one embodiment of the present invention, step III is conducted oxidatively, in particular through ozonolysis or by using hydrogen peroxide and sodium tungstate in catalytic amounts, more particularly by potassium permanganate/sodium hydroxide in tetrahydrofuran or by potassium permanganate/sodium hydroxide in water and small amounts of methylene chloride (see: 1 B. M. Cochran, Synlett 2016, 27, 245 for ozonolysis; 2 Jianwen Tan, Zhongyu Zhou, Jian Yan, Mei Zhang, Jing Wang, CN102432494 (A), 2012 for permanganate cleavage).

In one embodiment of the present invention, in step III, there is no temperature profile when adding potassium permanganate. In this embodiment the reaction mixture may be cooled to 0 °C but this is not obligatory. The product can be isolated, for example, via crystallization and extraction; in some embodiments of step III, crystallization is preferred in order to achieve a higher purity (see: Jianwen Tan, Zhongyu Zhou, Jian Yan, Mei Zhang, Jing Wang, CN102432494 (A), 2012 for Friedel-Crafts type reaction).

In one embodiment of the present invention, in step IV, the cyclization of the product to 4-carboxy-1-indanone is conducted through an intramolecular Friedel-Crafts-type acylation reaction. This reaction can be conducted in the presence of aluminum trichloride or, more preferably, in the presence of sulfuric acid as solvent and catalyst. In one specific embodiment of step IV, a continuous liquid-liquid extractor is used in order to achieve a high yield and a high purity (see: Jianwen Tan, Zhongyu Zhou, Jian Yan, Mei Zhang, Jing Wang, CN102432494 (A), 2012 for Friedel-Crafts type reaction).

In one embodiment of the present invention, in step V, the derivatization of the 4-carboxy-1-indanone to 4-cyano-1-indanone is carried out by forming a mixed anhydride by reacting the 4-carboxy-1-indanone with ethyl chloroformate, and then reacting the mixed acid species with ammonia as a nucleophile to yield a 4-amido-1-indanone and subsequent dehydration of the amide with conventional dehydration agents in a solvent. In a preferred embodiment, the dehydration reagents are selected from the group consisting of POCl₃, PCl₅, thionyl chloride, cyanuric chloride, phosphorous pentoxide, trifluoroacetic anhydride and mixtures thereof. A preferred dehydration reagent is trifluoroacetic anhydride. Particular examples of suitable solvents are toluene and/or dimethyl formamide (DMF) and/or acetonitrile (see: 1 a) S. Zhou, K. Junge, D. Addis, S. Das, M. Beller, Org. Lett. 2009, 11, 2461; b) F.-L. Yang, X. Zhu, D.-K. Rao, X.-N. Cao, K. Li, Y. Xu, X.-Q. Hao, M.-P. Song, RSC Adv 2016, 6, 37093; c) G. R. Hodges, WO 2012/123328; d) M. Mineno, Y. Sawai, K. Kanno, N. Sawada, H. Mizufune, The Journal of organic chemistry 2013, 78, 5843 for amide synthesis; 2 a) N. K. Bhattacharyya, Satadru Jha, Sangeeta Jha, Tshering Yangden Bhutia and Gita Adhikary, International Journal of Chemistry and Applications 2012, 4, 295; b) A. K. Yadav, V. P. Srivastava, L. D. S. Yadav, RSC Adv 2014, 4, 4181; c) Carr Albert A, Farr Robert, DE3031892 (A1), 1981 for dehydration).

In one embodiment of the present invention, in step VI, the conversion of the 4-cyano-1-indanone to 4-cyano-1-aminoindane in S-configuration is carried out via the racemate (see K. J. Gillen, J. Gillespie, C. Jamieson, J. K. F. Maclean, E. M. Moir, Z. Rankovic, WO 2010/115952).

In one embodiment of the present invention, in step VII, the protection of the amino-group of the 4-cyano-1-aminoindane is accomplished with a protecting agent. In general, any type of protecting group can be used. Overviews of suitable protecting reagents are given in the common standard textbooks of organic chemistry and are known to the person skilled in the art, such as from Theodora W. Greene "Protective Groups in Organic Synthesis".
Among preferred protecting agents are those selected from the group consisting of Boc (*tert*.-butyl-oxycarbonyl), Adoc (1-adamantyl-O(CO)NR₂), Alloc/Aloc (allylcarbamate), Cbz (benzyloxycarbonyl), cyclobutyloxycarbonyl, *N*-hydroxypiperinidyloxycarbonyl, *p*-nitrobenzyloxycarbonyl, 3,4-dimethoxy-6-nitrobenzyloxycarbonyl and 2,4-dichlorobenzyloxycarbonyl, with the Boc group being particularly preferred.

In one embodiment of the present invention, in step VIII, the conversion of the nitrile-group to an amidoxime group is carried out by the addition of hydroxylamine or the corresponding hydrochloride to the nitrile under basic (alkaline) conditions. Preferably, the basic conditions can be achieved by the presence of carbonates, particularly in the presence of short-chain alcohols for dissolving the substrates. In one further particular embodiment the alcohol is methanol and/or ethanol, and preferably under reflux (literature for step VIII-IX: a) R. Sheng, S. Li, G. Lin, S. Shangguan, Y. Gu, N. Qiu, J. Cao, Q. He, B. Yang, Y. Hu, RSC Adv 2015, 5, 81817; b) G. Schmidt, M. H. Bolli, C. Lescop, S. Abele, Org. Process Res. Dev. 2016, 20, 1637; c) M. R. Kuram, W. G. Kim, K. Myung, S. Y. Hong, Eur. J. Org. Chem. 2016, 2016, 438; d) K. S. Kadam, T. Gandhi, A. Gupte, A. K. Gangopadhyay, R. Sharma, Synthesis 2016, 48, 3996; e) X. Hou, J. Zhu, B.-C. Chen, S. H. Watterson, W. J. Pitts, A. J. Dyckman, P. H. Carter, A. Mathur, H. Zhang, Org. Process Res. Dev. 2016, 20, 989; f) D. Flesch, M. Gabler, A. Lill, R. C. Gomez, R. Steri, G. Schneider, H. Stark, M. Schubert-Zsilavecz, D. Merk, Bioorganic & Medicinal Chemistry 2015, 23, 3490; g) J. Cai, H. Wei, K. H. Hong, X. Wu, M. Cao, X. Zong, L. Li, C. Sun, J. Chen, M. Ji, European Journal of Medicinal Chemistry 2015, 96, 1; h) J. Boström, A. Hogner, A. Llinàs, E. Wellner, A. T. Plowright, J. Med. Chem. 2012, 55, 1817).

In one embodiment of the present invention, in step IX, the conversion of the amidoxime-group to a 1,4-oxadiazole heterocyclic-group is accomplished through the condensation with 3-cyano-4-isopropoxy benzoic acid or 3-cyano-4-isopropoxy benzoic acid derivative, particularly with 3-cyano-4-isopropoxy benzoic acid chloride. This step can be conducted in a solvent, such as in a polar aprotic solvent, preferably dimethylformamide (DMF), and in the presence of a coupling agent. Suitable coupling agents are known in the art and may be selected from the group consisting of hydroxybenzotriazole, carbodiimides such as 1-ethyl-3-(3-dimethylaminpropyl)-carbodiimide, dicyclohexylcarbodiimide (DCC), and ethylenediaminecarbodiimide (EDC), phosphonium reagents such as benzotriazolyloxytris(dimethylamino)-phosphonium hexafluorophosphate (BOP), Castro's Reagent (benzotriazol-1-yl-oxytripyrrolidinophosphoniumhexafluorophosphate, PyBOP), N-ethoxycarbonyl-2-ethoxy-1,2-dihydroquinoline (EEDQ), 4-(4,6-dimethoxy-1,3,5-triazin-2-yl)-4-methyl-morpholinium chloride (DMTMM), carbonyldiimidazole (CDI), 1-hydroxy-7-azabenzotriazole (HOAt), 1-[bis(dimethylamino)methylene]-1H-1,2,3-triazolo[4,5-b]pyridinium 3-oxid hexafluorophosphate (HATU), 2-(1H-benzotriazol-1-yl)-1,1,3,3-tetramethyluronium hexafluorophosphate (HBTU), hydroxy-3,4-dihydro-4-oxo-1,2,3-benzotriazine (HOOBt), and mixtures thereof.

In one embodiment of the present invention, in step X, the coupling of the protected amine with an epoxide is carried out. Following the addition of the hydroxyethyl group, the protecting group is cleaved under acidic conditions. Alternatively, an N-alkylation with compounds of general formula X-CH₂-CH₂-OH or -OSiR'₃ can be conducted, with X representing halogen and R' being selected from C₁ to C₄-alkyl, particularly methyl or ethyl.

The concepts underlying the alternative synthesis of Ozanimod according to the present invention are based on the amine (S)-2 as the key intermediate.

In addition, in one embodiment the bromo-substituted indanone can be utilized as a substrate for the preparation of the 4-substituted 1-aminoindane (preferably the 4-cyano-1-aminoindane compound (*S*)-2) prior to the specific biocatalytic methodologies.

After obtaining the desired amine (*S*)-2 from the indanone, the formation of the oxadiazole heterocycle represents one of the next steps towards the total synthesis of Ozanimod according to the present invention.

In another embodiment the present invention is directed to the preparation of the racemic amines which are used as the substrates for the resolution.

In context with the present invention, bio-transformations utilizing these racemic amine substrates are conducted.

The process of the present invention can be varied in several aspects *inter alia* in respect of:
- the biocatalyst (preferred are commercially available lipases),
- the reaction medium ("solvent engineering"),
- the acylation agent ("acyl donor", preferred are readily available bulk chemicals),
- reaction time and reaction temperature,
- high substrate loading (enabling high space-time-yield), and
- the indanon-1-amine substrates (bearing different 4-substituents).

### Preparation of the Racemic Amine Starting Materials

The racemic amines required as the starting materials for the enzymatic resolution step can be synthesized from the corresponding ketones by means of various methods.

A preferred synthetic method for the preparation of the racemic 4-substituted indan-1-amines is by applying a two-step protocol which consists of an initial condensation of a ketone with hydroxylamine under formation of a ketoxime and further reduction with zinc (as shown in the scheme below). As experimentally demonstrated herein, by means of this synthetic procedure, the desired racemic amines needed for the enzymatic resolution can be obtained in yields of up to 40 % or even more, depending on the specific conditions and substituents (R⁵ = H is not according to the invention): 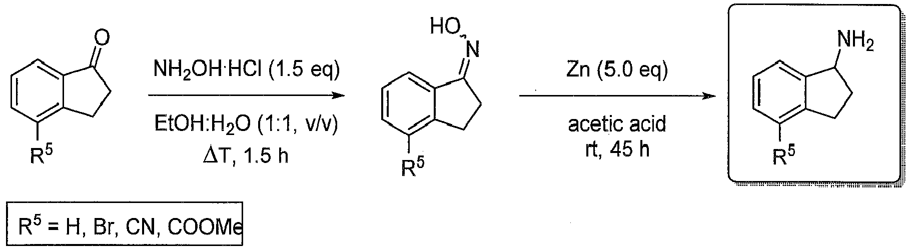 (Yield: H 37 % yield; Br 28 % yield; CN 35 % yield; COOMe 30 %, under the exemplary conditions mentioned in the reaction scheme above)

In principle, any commercially available lipase can be employed in the method according to the present invention. As noted above, preferred lipases are commercially available lipases, particularly those selected from the group consisting of lipase B from *Candida antarctica* (CAL-B), lipase from *Candida rugosa* (CR Lipase), lipase from *Burkholderia plantarii* (BPL) and lipase from *Burkholderia cepacia* (BC lipase). The lipase CAL-B is the most preferred biocatalyst according to the present invention, leading to significantly higher conversions (of up to 76 %) in comparison with the other two lipases.

In addition, various temperatures and different solvents can be employed. In one embodiment of the present invention an elevated temperature up to 90 °C, a temperature of 60 °C is preferably employed as the reaction temperature. The reaction temperature can be as low as 40 °C or even 10 °C.

As noted above, preferred organic solvents used as reaction medium are selected from the group consisting of *n*-heptane, methyl-*tert*.-butylether (MTBE), 2-methyltetrahydrofuran (2-MTHF), methylcyclohexane toluene and mixtures thereof, more preferably 2-MTHF and *n*-heptane, wherein *n*-heptane is particularly preferred.

With respect to the selection of the organic solvent as the reaction medium, in general and independent of the biocatalyst, it was found that the solvent *n-*heptane results in the highest conversions and thus represents the most preferred solvent. Further preferred solvents, in particular suitable for large scale applications, are selected from toluene, MTBE, 2-methyltetrahydrofuran, methylcyclohexane and *n*-heptane.

In addition, the acyl donor can be varied as well. Thus, the lipase-catalyzed resolutions, preferably CAL-B-catalyzed resolutions, were preferably carried out using an acyl donor selected from the group consisting of diethyl malonate (DEM), ethyl methoxyacetate (EMA), isopropyl methoxyacetate (IMA) and mixtures thereof, most preferably isopropyl methoxyacetate.

In one embodiment of the present invention, the solvent is 2-MTHF and the acyl donor is isopropyl methoxyacetate.

As further substrates, particularly the racemates of 4-cyano-1-aminoindane, 4-bromo-1-aminoindane and 4-methylcarboxylate-1-aminoindane can be used for in the resolution process according to the invention, using the lipase, preferably CAL-B.

Good E-values are obtained for all substrates. For the 4-cyano-substituted 1-aminoindane (i.e. compound (S)-2) the highest E-value of 31 is obtained, which leads to the formation of the desired remaining (*S*)-enantiomer of the amine (i.e. compound (*S*)-2) with an excellent enantiomeric excess of 99 % ee at a conversion of 58 %.

**Table 1. Conversions, ee-values and E-values for the enzymatic resolution of racemic 4-substituted 1-aminoindanes as Ozanimod precursors utilizing the lipase CAL-B (R⁶ = selected substituents of group R)**

| 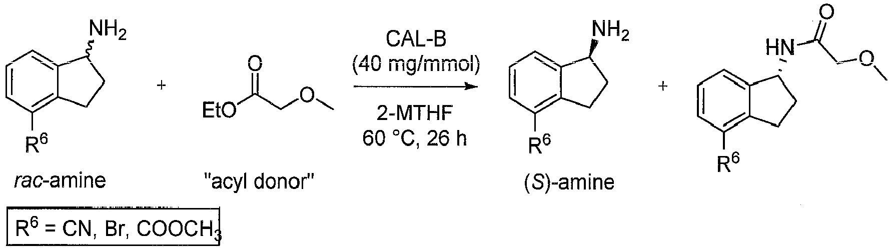 | | | | |
|---|---|---|---|---|
| R⁶ | Conversion [%] | *ee*_{P} [%] | *ee*_{S} [%] | E-value |
| Br | 59 | 68 | 99 | 26 |
| CN | 58 | 71 | 99 | 31 |
| COOCH₃ | 65 | 51 | 96 | 11 |

Since an attractive retro-synthetic approach for the preparation of Ozanimod is based on the racemic 4-carboxylated 1-aminoindane as a derivative from a multi-step route starting from naphthalene as an inexpensive raw material, resolution of this substrate is a preferred embodiment.

Thus, the process of the present invention (enzymatic resolution) represents a superior route for the preparation of the desired target intermediate (*S*)-4-cyano-1-aminoindane, as well as analogues with different 4-substituents thereof, leading to both good conversion as well as high enantioselectivity in the presence of a commercially available biocatalyst.

In one embodiment the process for the production of the key intermediate (*S*)-4-cyano-1-aminoindane can be a multi-step synthesis involving the lipase-catalyzed resolution as enantioselective reaction step. Besides the efficient resolution being suitable for the preparation of the desired (*S*)-4-cyano-1-aminoindane, the present invention also provides a suitable approach towards the racemic substrate and the combination of it with the resolution step according to the invention, followed by a suitable work-up under isolation of this (*S*)-amine as the target compound and intermediate for further derivatization towards Ozanimod.
This multi-step synthesis of the target amine (*S*)-4-cyano-1-aminoindane is illustrated through a particular, but non-limiting example, as follows: The substrate synthesis starts from the corresponding indanone and two-step conversion into the racemic amine based on oxime-formation and reduction to furnish the racemic amine *rac-2.* Subsequently, the lipase-catalyzed resolution according to invention route A is conducted, followed by separation from the *N*-acylated product. Subsequent purification comprising the steps acidic extraction and neutralization then furnishes the desired chiral (*S*)-4-cyano-1-aminoindane enantiomer.

In addition, it is worth mentioning that the enantioselectivity of the enzymatic resolution process in the synthesis of the desired (*S*)-4-cyano-1-aminoindane can be enhanced further, if the isopropyl ester is used instead of the methyl ester of the methoxyacetate acyl donor, making the isopropyl methoxyacetate the preferred ester according to the present invention. Accordingly, in a preferred embodiment of the invention, isopropyl methoxyacetate is used as the acyl donor in the method for resolving the chiral mixture of 4-cyano-1-aminoindane (*rac*-2).

The preferred use of the lipase in immobilized form and using an organic solvent as the reaction medium makes it easier to separate the catalyst from the reaction medium since the biocatalyst can be separated by a simple filtration. Thus, the recycling of the lipase is easier.

Accordingly, also the recycling of the recovered lipase, preferably CAL-B, is part of the present invention.

It is noteworthy that the conversion remains high and nearly unchanged even after the fifth reaction cycle, illustrating the high rate of recyclability of the lipase.

The amount (mg) of enzyme per mmol substrate can range from 1 mg/mmol to 100 mg/mmol, from 1 mg/mmol to 75 mg/mmol, from 1 mg/mmol to 50 mg/mmol, from 5 mg/mmol to 40 mg/mmol, or from 10 mg/mmol to 20 mg/mmol. An amount of, for example, 40 mg of immobilized lipase, e.g. CAL-B, per mmol of substrate can be used in the bio-transformations. However, the amount can also be reduced to 20 mg/mmol, 10 mg/mmol and 5 mg/mmol enzyme per mmol substrate, respectively; or any other desired amount.

Independent of the amount of the catalyst, the reaction proceeds smoothly in all cases. As expected, the reaction rate is decreasing, if the amount of lipase is reduced. If using increased amounts of lipase, e.g. CAL-B, per mmol of substrate, after some time nearly the same ee-values can be achieved. This means that in all of these bio-transformations the reaction is close to completion. If using lower amounts of lipase per mmol of substrate, the ee-value after comparable time is either still lower but the reaction is still in progress, or it is about the same. At a prolonged reaction time, the reaction will also reach completion.

Accordingly, low catalyst loadings can be used, if costs are a factor; the results are ultimately the same, only the duration until it is reached is different.

Usually, the reaction is conducted within a time-period of 0.5 hours to 24 hours, but can be adapted to shorter or longer reaction times depending on the actual requirements/desires.

Due to the effectivity of the process of the present invention and the recyclability of the lipase, attractive biocatalyst costs per kilogram of product (*S*)-4-cyano-1-aminoindane ((*S*)-2) can be achieved.

Accordingly, of the presented four routes A), B), C) and D), the lipase-based route A) represents the preferred route of the present invention.

In another aspect, the invention is directed to the total synthesis of Ozanimod. Based on the various asymmetric biocatalytic reactions as key steps in the synthesis of the chiral amine intermediate (*S*)-4-cyano-1-aminoindane and with the preferred lipase-catalyzed resolution enabling an efficient synthesis of this amine in hand, a total synthesis of Ozanimod starting from a readily available and inexpensive bulk chemical as a starting material also represents an embodiment of the present invention.

Accordingly, it has been an additional object of the present invention to identify simple reaction steps having a technical potential for the synthesis of the substrate for the resolution step.

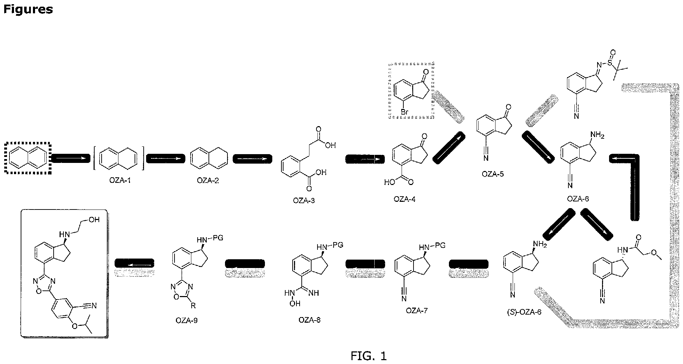

A particular focus for the optimization of the overall synthetic route towards Ozanimod according to the present invention centers on an alternative approach for providing the 4-substituted 1-aminoindanes, such as the key building block (*S*)-4-cyano-1-aminoindane mentioned above. The concept of the novel, alternative route being subject of this invention is shown as the dark pathway in Figure 1 (while the pathway of WO 2015/06615 is light grey).

Two major key issues have been addressed by the present invention. On the one hand, the stereogenic center is introduced by means of an attractive biocatalytic approach.
The second key issue is related to the synthesis of the 4-cyano-indan-1-one, other related 4-substituted analogues as well as their corresponding racemic amines (for the lipase-catalyzed resolution approach), which then serve as substrates for the biocatalytic step. In the present invention, these compounds were synthesized from inexpensive starting raw materials and by means of well-established chemical reactions.

In the total synthesis of Ozanimod, naphthalene is a raw material of interest due to its large-scale availability at low cost. In a first step, naphthalene can be reduced to 1,2-dihydro-naphthalene.
Next, the C=C-double bond can be oxidatively cleaved resulting in a dicarboxylic acid, followed by cyclization forming the corresponding indanone, for example through a Friedel-Crafts-reaction. Subsequently, the remaining carboxylic acid moiety in the indanone is derivatized to a nitrile group. The corresponding racemic amines are converted via the lipase-based process of the present invention. The undesired enantiomer from this step can preferably be acylated and subsequently racemized and, thus, recycled.

With the key intermediate (*S*)-4-cyano-1-aminoindane in hand, the further synthetic steps towards Ozanimod are analogous to the route of WO 2015/066515 consisting of the formation of the oxadiazole heterocycle and final introduction of the N-substituent.

Naphthalene can be reduced and rearranged towards 1,2-dihydronaphthalene. In one alternative of the present invention, the reduction of naphthalene is preferably carried out electrochemically.

In another alternative of the present invention towards the desired 1,2-dihydronaphthalene, the Birch reaction is chosen as an initial step.

Naphthalene reacts in the presence of sodium and tert.-BuOH under the formation of 1,4-dihydronaphthalene, which can be obtained in high yields, in one preferred embodiment 80 % yield or more, even more preferred 85 % yield or more.
Since the undesired 1,4-product is usually the major regio-isomer, a subsequent rearrangement to the required 1,2-dihydronaphthalene can be performed. This reaction can be based on the formation of an ion either by a strong base (deprotonation) or by a strong acid (protonation). It is preferred to use bases for the rearrangement reaction.
The base-catalyzed rearrangement can be performed by varying the concentration of the base and the nature of the solvent. Generally, all kinds of bases (and catalysts) can be used here.
The amount of base can be varied widely, for example from 0.01 eq to 1 eq; and the temperature can also be widely varied, for example between 20 °C and 100 °C.
Preferably, the rearrangement process according to the present invention employs a combination of potassium *tert*.-butanolate and *tert*.-BuOH, leading to a ratio of the two regio-isomers of 97:3 with a preference for the desired 1,2-dihydronaphthalene. Particularly this rearrangement is carried out at a temperature of 40 to 80 °C and with 0.2 to 0.6 eq. of base, preferably at 55 to 65 °C and with 0.35 to 0.45 eq base.

The C=C-double bond can oxidatively be cleaved selectively into 1,2-dihydronaphthalene. In order to obtain the 4-carboxy-substituted indan-1-one, a selective cleavage of the corresponding C=C-double bond of 1,2-dihydronaphthalene is carried out.
This cleavage can be carried out, for example, through ozonolysis or the oxidation with hydrogen peroxide and sodium tungstate in catalytic amounts. Particularly preferred is an oxidation by using potassium permanganate as this leads to improved results and very high conversions at high yields. In one particular embodiment, this reaction is conducted in the presence of sodium hydroxide. In addition, good selectivity indicated by a lack of observed by-product formation as well as simple product isolation are further advantages.

Using this method the desired product is formed and obtained in yields of 70 % or more, particularly 75 % or more within a short reaction-time of preferably 2 to 4 hours.

The yield can be controlled by the amount of water present, which influences the precipitation of the product. Alternatively, the product can be also isolated by means of extraction.

As a next step, the dicarboxylic acid obtained by oxidative C=C-double bond cleavage is converted into the 4-carboxy-substituted indane-1-one. In a preferred embodiment, this conversion is carried out by means of an intramolecular *Friedel-Crafts* acylation. While the "classic" approach by converting both carboxylic acid functional groups into the corresponding acid chloride by addition of aluminum chloride can be used, this is less preferred. Similarly feasible is an approach in which sulfuric acid as a very inexpensive Brønsted acid catalyst is used as both solvent and catalyst. Reaction temperatures can be between 100 and 180 °C, preferably 100 to 160 °C and particularly 125 to 135 °C. The raw product can be extracted, particularly three times or it can be precipitated directly from the sulfuric acid solution to increase yield and purity.

Instead of using the inexpensive sulfuric acid as a catalyst in the synthesis, alternative catalytic systems such as heterogeneous Lewis acid or Brønsted acid catalysts, which simplify work-up, can be employed in accordance with the present invention.

For the desired conversion of the 4-carboxy group of the indan-1-one derivative into 4-cyanoindanone, at first the conversion of the carboxylic acid substituent into a primary amide group is performed.

A mixed anhydride from the reaction of the acid with ethyl chloroformate is formed, which then reacts as an activated acid species with ammonia as nucleophilic. In the conversion to the anhydride, the equivalent amount of ethyl chloroformate should be at least 1.5, preferably higher than 4.0. A full conversion is achieved when further increasing the stoichiometric amount up to 5.0 eq of ethyl chloroformate, leading to the mixed anhydride as a crude product in yields of 80 % or more, particularly 85 % or more.

The formation of the amide is then carried out by adding an aqueous ammonia solution at room temperature. Increasing the reaction temperature to 60 °C leads to a quantitative conversion and the desired amide can be obtained in a yield of 65 % or more, preferably 70 % or more of the crude product.

In the subsequent dehydration of the amide under the formation of the nitrile (OZA-5), various reagents which are suitable for dehydration reactions of amides such as various phosphorus compounds, such as POCl₃, PCl₅, P₂O₅, thionyl chloride, trifluoroacetic anhydride as well as cyanuric chloride can be used. The dehydration works in principle with all reagents. The yields, however, differ from each other. At the same time it is noteworthy that no starting material can be detected any more, thus indicating full conversion. Phosphorus pentoxide and trifluoroacetic anhydride are preferred dehydrating reagents in terms of performance and suitability for handling.

A recovery of 80 % of the mass used at the start of the reaction can be achieved.
It would appear that the ratio of amide to anhydride does not change significantly. The anhydride is hydrolyzed during the basic extraction and remains in aqueous phase. Thus, it can be detected in the product mixture at a lower amount.

Prior to the enzymatic resolution, the indanone moiety is converted into the corresponding racemic indan-1-amine. Reductive amination of ketones to primary amines is still a challenge in industry. The reason is the lability of the primary imine, which is formed as an intermediate.

In the conversion of the ketone to the primary amine, the Leuckard-Wallach chemistry can be employed in which ammonium formate is used as a source of nitrogen and the oxidation of formic acid to carbon dioxide, which escapes as a gas, represents the driving force of this reaction.

Another approach is the dehydration of the readily available ketoxime, which is formed in a condensation reaction of the indan-1-one with hydroxylamine.

Utilizing 4-cyanoindanone as the ketone component, the resulting ketoxime can be isolated in quantitative yield. The subsequent reductive conversion into the desired amine then takes place in the presence of acetic acid and by means of a reducing agent, preferably zinc.

The enzymatic (kinetic) resolution of racemic indan-1-amines (being unsubstituted and 4-substituted) turned out to be the preferred enantioselective route towards the (S)-enantiomer of 4-cyano-indan-1-amine, as a key intermediate, as described above.

In general, per definition, kinetic resolutions are limited to a maximum theoretical yield of 50 % for the desired enantiomer. In the case of a lower enantio-selectivity of the catalyst, the yield for the enantiomerically highly enriched compound (either as product or remaining substrate) can be significantly lower.

The unwanted enantiomer resulting from the resolution step, which is not relevant for the synthesis of the active ingredient, is recycled and re-used in the process after racemization. This is of particular economical relevance.

In the racemization of primary chiral amines, two alternative routes exist. One possibility is analogous to the redox racemization of secondary alcohols with a transition metal catalyst. However, in this case a large amount of catalyst loading is often required and the high price for, e.g., the Shvo (ruthenium) catalyst limits the economic attractiveness of this approach.

Another alternative route preferred in the present invention proceeds as follows: Initially, a Schiff base is formed from the amine by means of a condensation reaction with the corresponding ketone. The resulting imine is subsequently deprotonated with another strong base.

Through the fully conjugated system, the negative charge can be distributed over the entire molecule, and the former stereogenic center is temporarily hybridized to sp². Without chiral information from other substances, the absolute conformation of the stereogenic center is destroyed, thus forming the corresponding racemic form.

Since the variation of the base and the equivalents of ketone have an influence on the rate of racemization, optimized kinds of base and equivalents of ketones are used. In a preferred embodiment carbonyl compounds, in particular ketones and aldehydes, particularly 3,5-dinitrosalycylaldehyde, with acceptor properties are used since they lead to an increased reaction rate.

### Amidoxime Formation

The oxadiazole forms one of the last molecular structures in the overall reaction to Ozanimod. It must be prepared by the addition of hydroxylamine (or hydroxylammonium chloride) to a nitrile, followed by the oxadiazole ring formation through the condensation of the amidoxime species with a carboxylic acid derivative.
The amidoxime is formed under basic conditions and usually carbonates are used. Preferred solvents are short-chain alcohols, such as methanol or ethanol, which are heated to reflux during the reaction.

By varying base and hydroxylamine equivalents, the by-product formation can be influenced. The amide formation can also be changed by the temperature. For example, lowering the temperature to 25 °C results in less by-product formation.

An increased amount of base results in a higher conversion to the desired product as well as in a faster reaction.

A reaction parameter having a strong influence on amide formation is the concentration of hydroxylamine or the corresponding hydrochloride thereof during the reaction.

Thus, controlling the dosage of hydroxylamine is a preferred strategy to suppress the formation of the undesired amide by-product.

In the oxadiazole condensation the amidoxime can be condensed with an aromatic acid chloride. This reaction can be conducted to proceed quantitatively or nearly quantitatively, leading to the desired oxadiazole in high yield.

The various embodiments of the present invention are not to be construed to be limited to the embodiments set forth in the claims and examples but can be combined in any suitable manner.
The present invention will now be explained further by the following non-limiting examples. These examples are non-exhaustive and are not intended to limit the scope of the invention. Although the present invention has been described with reference to particular embodiments, it should be recognized that these embodiments are merely illustrative of the principles of the present invention. Those of ordinary skill in the art will appreciate that the compositions, apparatus and methods of the present invention may be constructed and implemented in other ways and embodiments. Accordingly, the description herein should not be read as limiting the present invention, as other embodiments also fall within the scope of the present invention as defined by the appended claims.

### Examples

### Experimental Protocols and Data related to Route A: Resolution of a Racemic Amine via Acylation with a Lipase as the Key Step

### Derivatization of Amines for Analytical Purpose (HPLC)

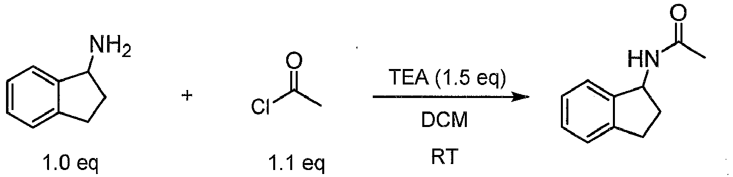

The sample was acetylated with acetyl chloride (1.1 eq) and triethylamine (1.5 eq) in methylene chloride for one hour. The suspension was washed with a solution of hydrogen chloride (1:1, v/v). The solvent was removed *in vacuo.* Enantiomeric excess and conversion were determined via HPLC.
For the determination of the conversion of the lipase-catalyzed acylation reactions, at first ¹H-NMR spectroscopy was used. Therefore, after separation of the reaction mixture from the enzyme and removal of the solvent in *vacuo,* the crude product was subjected to an ¹H-NMR analysis, and in the resulting ¹H-NMR spectrum the benzylic protons at the amino- and *N*-acylamino-functionalized carbon-atoms of substrate (triplet) and product (quartet), respectively, were used and compared (quartet at 5.54 ppm: *rac*-1-indanylacetamid, triplet at 4.37 ppm: *rac*-1-aminoindane).

For the determination of the enantiomeric excess by means of chiral HPLC analysis, the extracted crude product can be isolated and derivatized *in situ* without further workup. The acylation component in this derivatization step differs from the one used in the resolution, thus leading to two different types of amides. This derivatization step can sometimes be necessary due to possible difficulties in determining the ee-value directly from the non-derivatized amine.

From the resulting chiral HPLC-chromatogram, which shows a clear separation of all four amide enantiomers (from which two are related to the product and two to the substrate from the resolution step), the ee values for remaining substrate and formed product in the lipase-catalyzed resolution can be directly calculated. Furthermore, the conversion and the E-value (enantioselectivity value) can be calculated also from these ee-values.

### Initial Experiments with Various Lipases, Temperatures and Solvents were Conducted Showing these Findings:

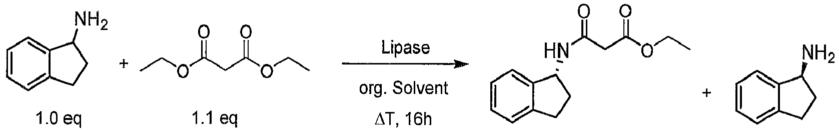

**Table 2: Initial experiments.**

| Enzyme | T/°C | Solvent | Conversion [%] |
|---|---|---|---|
| CAL-B | 40 | *n*-heptane | 6 |
| CAL-B | 60 | *n*-heptane | 74 |
| CAL-B | 40 | MTBE | 20 |
| CAL-B | 60 | MTBE | 67 |
| BC | 40 | toluene | 0 |
| BC | 60 | toluene | 4 |
| BC | 60 | *n*-heptane | 24 |
| BC | 60 | MTBE | 3 |
| CR | 40 | toluene | 0 |
| CR | 60 | toluene | 7 |
| CR | 60 | *n*-heptane | 9 |
| CR | 60 | MTBE | 2 |

In addition, the commercially available lipase PS was tested as an alternative lipase for the racemization of 1-aminoindane. In our experiments lipase PS and lipase PS-SD (SD means diluted with dextrin) were tested. A low conversion with 1-aminoindane was shown with both enzymes. However, 4-cyano-1-amino-indane (**2**) did not turn out as a suitable substrate for the lipase PS.

### Two-step Reductive Amination for the Synthesis of Racemic Amines

4-cyano-1-indanon (1 eq.) and hydroxylamine hydrochloride (1.5 eq.) were dissolved in ethanol and water (1:1, v/v). Meanwhile sodium hydroxide (1.75 eq) dissolved in water was added to the suspension. The mixture was heated to reflux for 90 minutes. The crude product was filtered over celite and washed with water. The solvent was removed *in vacuo.* The oxime was dissolved in acetic acid. Under argon atmosphere zinc-dust (5 eq.) was added and the suspension was stirred at room temperature for 48 hours. The reaction mixture was filtered over celite, washed with ethyl acetate and the solvent was removed *in vacuo.* The oil was dissolved in ethyl acetate and hydrogen chloride (1:1, v/v) and extracted twice with 2 M hydrogen chloride. The pH value of the aqueous phase was adjusted to 10 and afterwards extracted three times ethyl acetate. The organic phase was washed with brine and dried over MgSO₄. The solvent was removed *in vacuo.* The remaining solvent was removed in a *Schlenk-flask* under argon atmosphere.

### Experimental Data for rac-1-Aminoindane

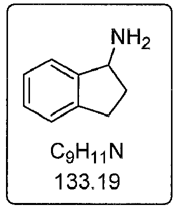

**Yield:** 0.18 g (1.38 mmol, 37%).
**¹H-NMR** (500 MHz, CDCl3):*δ*[ppm] =1.70-1.76 (m, 1H, C**H**₂), 2.51-2.56 (m, 1H, C**H**₂), 2.83-2.87 (m, 1H, C**H**₂), 2.95-3.02 (m, 1H, C**H**₂), 4.37 (t, ³*J*= 7.5 Hz, 1H, NH₂C**H**), 7.19-7.25 (m, 3H, Ar**H**), 7.33-7.35 (m, 1H, Ar**H**).
**MS:** m/z = 134.5 [M+H]⁺.

### Experimental Data for rac-4-Bromo-1-Aminoindane

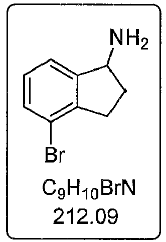

**Yield:** 0.36 g (1.68 mmol, 28 %).
**¹H-NMR** (500 MHz, CDCl3): *δ*[ppm] = 1.61-1.64 (m, 1H, C**H**₂), 2.42-2.50 (m, 1H, C**H**₂), 2.69-2.75 (m, 1H, C**H**₂), 2.91-2.97 (m, 1H, C**H**₂), 4.33 (t, ³*J*= 7.5 Hz, 1H,NH₂C**H**), 7.00 (t, ³*J*= 7.6 Hz, H, Ar*ₘ***H**), 7.17 (d, ³*J*= 7.4 Hz, 1H, Ar_{*o*/*p*} **H**), 7.27 (d, ³*J* = 7.9 Hz, 1H, Ar_{*o*/*p*} **H**).
¹³**C-NMR** (500 MHz, CDCl3): *δ*[ppm] = 144.2 (CH-**C**-CH2), 143.1 (CH-**C-**CH2), 131.9 (Ar-**C**), 129.0 (Ar-**C**), 123.5 (Ar-**C**), 120.4 (Ar-**C**), 56.8 (N-**C**H₂), 31.8 (**C**H₂),31.6 (**C**H₂).
**MS** (ESI): m/z = 211.9 [M+H]⁺, 213.9 [M+H]⁺

### Experimental Data for rac-4-Cyano-1-Aminoindane

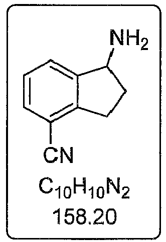

**Yield:** 28 mg (0.18 mmol, 35 %).
**¹H-NMR** (500 MHz, CDCl3): *δ*[ppm] = 2.11-2.17 (m, 1H, C**H**₂), 2.60-2.69 (m,1H, C**H**₂), 3.05-3.10 (m, 1H, C**H**₂), 3.28-3.33 (m, 1H, C**H**₂), 4.65 (t, ³*J*= 7.5 Hz, 1H, NH₂C**H**), 7.37 (t, ³*J*= 7.6 Hz, 1H, Ar*ₘ***H**), 7.56 (d, ³*J*= 7.4 Hz, 1H, Ar_{*o*/*p*} **H**), 7.70 (d, ³*J*= 7.9 Hz, 1H, Ar_{*o*/*p*} **H**).
¹³**C-NMR** (500 MHz, CDCl3): *δ*[ppm] = 148.2 (CH-**C**-CH₂), 141.7 (CH-**C**-CH₂), 132.6 (Ar-**C**), 129.6 (Ar-**C**), 126.1 (Ar-**C**), 117.3 (**C≡**N) 109.5 (Ar-**C**), 55.8 (N-**C**H₂), 31.3 (**C**H₂), 30.0 (**C**H₂).
**MS** (ESI): m/z = 159.0 [M+H]⁺.

### Experimental Data for rac-4-Methylcarboxylat-1-Aminoindane

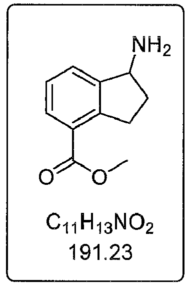

**Yield:** 0.30 g (1.57 mmol, 30 %).
¹**H-NMR** (500 MHz, CDCl₃) *δ*/ ppm: 1.69-1.76 (m, 1H, C**H**₂), 2.55-2.61 (m, 1H, C**H**₂), 3.10-3.17 (m, 1H, C**H**₂), 3.37 (s, 3H, C**H**₃), 3.45-3.53 (m, 1H, C**H**₂), 4.39 (t, ³*J*= 7.5Hz, 1H, NH₂C**H**), 7.32 (t, ³*J*= 7.6 Hz, 1H, Ar*ₘ***H**), 7.54 (d, ³*J*= 7.4 Hz, 1H, Ar_{*o*/*p*} **H**), 7.91 (d, ³*J*= 7.9 Hz, 1H, Ar_{*o*/*p*} **H**).

### Resolution of 1-Aminoindane Catalyzed by Lipases

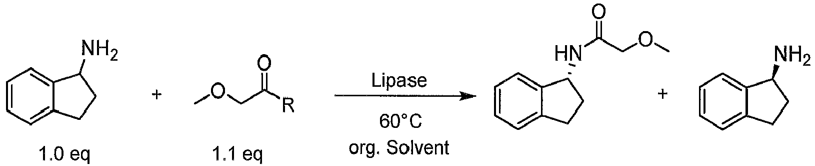

1-Aminoindane (1.0 eq.) and the acyl-donor (1.1 eq.) were dissolved in organic solvent. Lipase was added and heated to 60°C. At fixed times, samples were taken. This sample were acetylated with acetyl chloride (1.1 eq.) and triethylamine *(1.5* eq.) in methylene chloride for one hour. The suspension was washed with hydrogen chloride (1:1, v/v). The solvent was removed *in vacuo.* Enantiomeric excess and conversion were determined via HPLC.

### Initial Experiments for Kinetic Resolution with Various Lipases

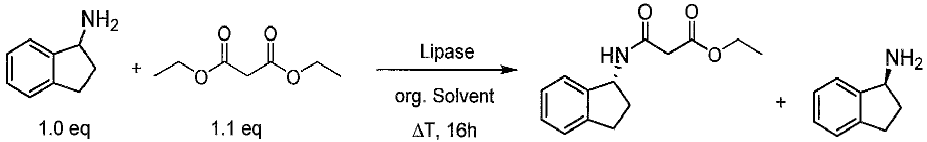

For the initial experiments, 1-aminoindane (20 mg, 0.15 mmol) was dissolved with diethyl malonate (24 mg, 0.20 mmol) in organic solvent (256 µL) in *Eppendorf* vessels. Lipase was added to the reaction solution (6 mg). The reaction solution was stirred for 16 hours at appropriate temperature. Subsequently, the enzyme was filtered off and washed with dichloromethane. The solvent was removed *in vacuo.* Conversion was determined by ¹H NMR spectroscopy.

### Solvent-, Acyl Donor-, Temperature Screening of CAL-B-Catalyzed Reactions

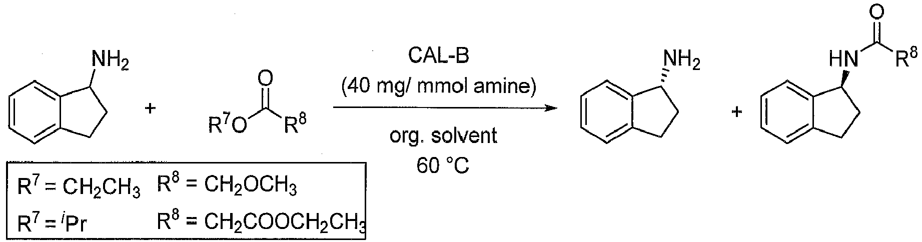

For determination of optimal solvent, 1-aminoindane (30 mg, 0.22 mmol) and diethyl malonate (40 mg, 0.25 mmol) or ethyl methoxyacetate, (29 mg, 0.29 mmol) were dissolved in the solvent (384 µl) and CAL-B (9 mg) was added. The reactions were carried out in screw cap glasses of 5 mL. The following solvents were tested, toluene, MTBE, 2-methyltetrahydrofuran (2-MTHF), methylcyclohexane (MCH) and *n*-heptane. The reaction with isopropyl methoxy acetate (25 mg, 0.19 mmol) was carried in 2-methyltetrahydrofuran (384 µl). Samples were taken after 6, 26 and 48 hours. The enzyme was filtered and washed with dichloromethane. The samples were acylated. The conversion was carried out using ¹H-NMR spectroscopy and HPLC. The enantiomeric excess was determined via chiral HPLC.

Results for Solvent, Acyl-Donor, Temperature Screening of CAL-B-Catalyzed Reactions are shown in the following Table 3:

**Table 3**

| Solvent | Acyl donor | T/°C | t/h | Conv./%^{a} | Conv./%^{b} | *ee_{P}*/*%^{c}* | *ee_{S}*/*%^{d}* | E-value^{e} |
|---|---|---|---|---|---|---|---|---|
| 2-MTHFⁱ | DEM^{f} | 60 | 6 | 53 | 50 | 82 | 91 | 32 |
| 2-MTHFⁱ | DEM^{f} | 60 | 26 | 59 | 59 | 70 | 99 | 28 |
| Toluene | DEM^{f} | 60 | 6 | 55 | 53 | 80 | 96 | 36 |
| Toluene | DEM^{f} | 60 | 26 | 55 | 60 | 79 | 97 | 35 |
| MTBE | DEM^{f} | 60 | 6 | | 46 | 82 | 69 | 19 |
| MTBE | DEM^{f} | 60 | 26 | | 54 | 81 | 95 | 20 |
| MCH^{j} | DEM^{f} | 60 | 6 | 53 | 49 | 83 | 95 | 40 |
| MCH^{j} | DEM^{f} | 60 | 26 | 58 | 55 | 70 | 98 | 25 |
| Toluene | DEM^{f} | 80 | 6 | 54 | 49 | 82 | 98 | 46 |
| Toluene | DEM^{f} | 80 | 26 | 65 | 53 | 54 | 99 | 16 |
| n-heptane | DEM^{f} | 60 | 6 | | 50 | 70 | 73 | 12 |
| n-heptane | DEM^{f} | 60 | 26 | | 57 | 59 | 77 | 9 |
| 2-MTHFⁱ | EMA^{g} | 60 | 6 | 55 | 55 | 81 | 99 | 52 |
| 2-MTHFⁱ | EMA^{g} | 60 | 26 | 59 | 58 | 72 | 99 | 31 |
| Toluene | EMA^{g} | 60 | 6 | 57 | 56 | 77 | 97 | 31 |
| Toluene | EMA^{g} | 60 | 26 | 65 | 59 | 70 | 99 | 28 |
| MTBE | EMA^{g} | 60 | 6 | 67 | 68 | 66 | 97 | 20 |
| MTBE | EMA^{g} | 60 | 26 | 70 | 70 | 59 | 99 | 19 |
| MCH^{j} | EMA^{g} | 60 | 6 | 55 | 54 | 81 | 97 | 39 |
| MCH^{j} | EMA^{g} | 60 | 26 | 60 | 60 | 67 | 99 | 25 |
| 2-MTHFⁱ | EMA^{g} | 80 | 6 | 55 | 55 | 81 | 99 | 49 |
| 2-MTHFⁱ | EMA^{g} | 80 | 26 | 69 | 56 | 79 | 99 | 44 |
| n-heptane | DEM^{f} | 60 | 6 | | 53 | 87 | 98 | 56 |
| n-heptane | DEM^{f} | 60 | 26 | | 54 | 83 | 99 | 58 |
| 2-MTHF | IMA^{h} | 60 | 6 | | 41 | 97 | 68 | 14 |
| 2-MTHF | IMA^{h} | 60 | 26 | | 43 | 95 | 72 | 84 |

| | | | | | | | | |
|---|---|---|---|---|---|---|---|---|
| ^{a}conv. *via* ¹H-NMR; ^{b}conv. *via* HPLC; ^{c}calc. *via* HPLC-data; ^{d}calc. *via* HPLC-data; ^{e}calc. *via ee* data; ^{f}diethyl malonate, ^{g}ethyl methoxyacetate; ^{h}isopropyl methoxyacetate; ⁱ2-methyl-THF; ^{j}methylcyclohexane (MCH) | | | | | | | | |

### Acyl Donor and Solvent Screening at 60 °C for the Resolution of Indan-1-Amine with Lipase CAL-B

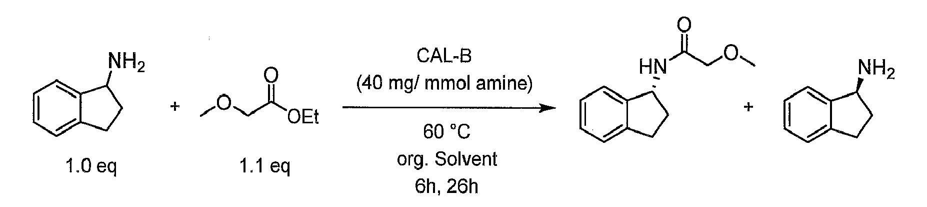

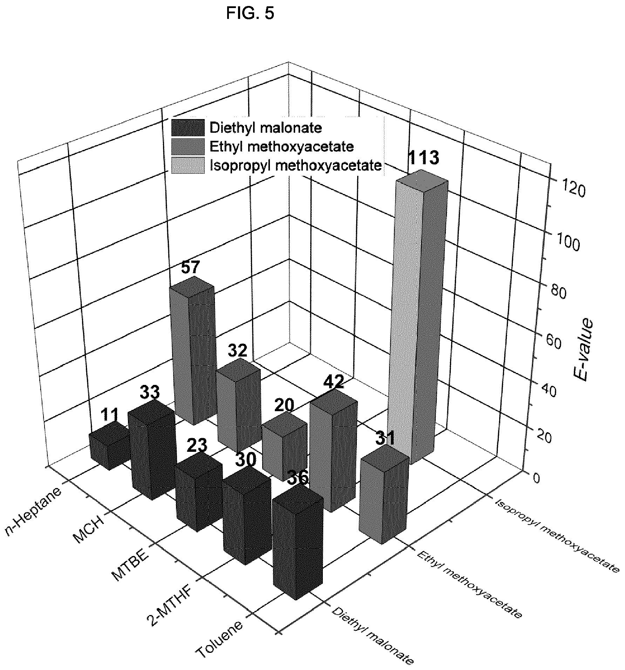

Although the highest E-value was achieved when utilizing the isopropyl ester of methoxyacetate with E=113, the utilization of ethyl methoxyacetate as well as diethyl malonate also led to good and synthetically still attractive E-values of up to 57 and 36, respectively. With respect to the solvent component, in general MTBE led to somewhat lower E-values compared to 2-methylhydrofuran and toluene. It is noteworthy that with n-heptane as a solvent, a high E-value with E=57 was achieved when using ethyl methoxyacetate as an acyl donor whereas the use of diethyl malonate led to the lowest observed E-value for this acyl donor with E=11 only. See in this context also Figure 5.

### CAL-B-Catalyzed Reaction with 1M Substrate Loading

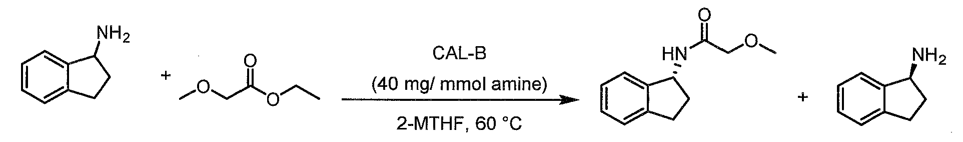

For determination of CAL-B-catalyzed reaction with 1 M substrate loading 1-aminoindan (665 mg, 5.03 mmol), ethyl methoxyacetate (770 mg, 7.09 mmol) and CAL-B (0.20 g) were dissolved in 2-methyltetrahydrofuran (5 mL). The reaction was stirred at 60 °C. Samples were taken after 6 and 26 hours. The enzyme was filtered off and washed with dichloromethane. The samples were acylated. The conversion was carried out using ¹H-NMR spectroscopy and HPLC. The enantiomeric excess was determined via chiral HPLC.

Results for CAL-B-Catalyzed Reaction with 1 M Substrate Loading:

**Table 4**

| *t*/*h* | *conv.*/*%^{a}* | *ee_{P}*/*%^{b}* | *ee_{S}*/*%^{b}* | *E-value* |
|---|---|---|---|---|
| *6* | *47* | *89* | *80* | *42* |
| *26* | *56* | *73* | *94* | *22* |

| | | | | |
|---|---|---|---|---|
| ^{a} conv. via HPLC; ^{b} calc. via HPLC | | | | |

For evaluation whether the CAL-B-catalyzed kinetic resolution is also possible in high substrate concentrations without causing inhibition effects, a reaction with 1 M substrate was performed. Therefore, the E-value of the reaction running at analytical scale with a concentration of 0.57 M was compared with the one of a biotransformation running at an elevated substrate loading of 1 M, showing that similar E-values are obtained independent of the substrate concentration.

### CAL-B-Catalyzed Reaction with 1-Aminoindane Derivatives

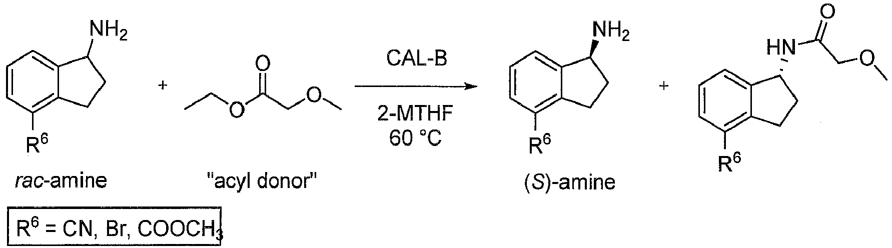

For the CAL-B-catalyzed reactions of 1-aminoindane derivatives (1.0 eq.) were dissolved in appropriate solvent with acyl donor (1.1 eq.). The reactions are carried out in screw cap glasses with a volume of 5 mL. CAL-B (40 mg/ mmol amine) was added to the reaction solution. After 26 hours, a sample was taken and derivatized. Subsequently, the enzyme was filtered and washed with dichloromethane. The conversion and the enantiomeric excess were determined by means of chiral HPLC.

| | | | | |
|---|---|---|---|---|
| R⁶ | Conversion [%] | ee_{P} [%] | eeₛ [%] | E-value |
| Br | 59 | 68 | 99 | 26 |
| CN | 58 | 71 | 99 | 31 |
| COOH₃ | 65 | 51 | 96 | 11 |

### CAL-B-catalyzed Reaction with 4-Cyano-1-Aminoindane

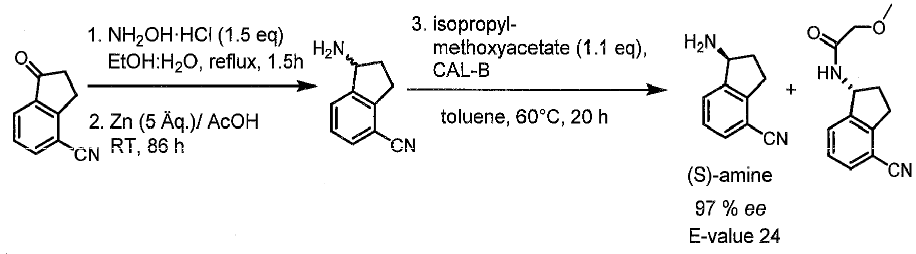

4-Cyano-1-indanon (0.40 g, 2.55 mmol) and hydroxylamino hydrochloride (0.27 g, 3.84 mmol) were dissolved in 10 mL ethanol and water (1:1, v/v). Meanwhile sodium hydroxide (0.17 g, 4.51 mmol) dissolved in 1 mL water was added to the suspension. The mixture was heated to reflux for 90 minutes. The crude product was filtered over celite and washed with water. The solvent was removed *in vacuo.* The oxime was dissolved in 10 mL acetic acid. Under argon atmosphere zinc-dust (0.83 g, 12.77 mmol) was added and the suspension was stirred at room temperature for 86 hours. The reaction mixture was filtered over celite, washed with ethyl acetate and the solvent was removed *in vacuo.* The oil were dissolved in 10 mL ethyl acetate and hydrogen chloride (1:1, v/v) and extracted with 2 M hydrogen chloride (2x10 mL). The pH value of the aqueous phase was adjusted to 10 and afterwards extracted with ethyl acetate (3x10 mL). The organic phase was washed with brine and dried over MgSO₄. The solvent was removed *in vacuo.* The remaining solvent was removed in a *Schlenk*-flask under argon atmosphere.

4-Cyano-1-aminoindan (0.14 g, 0.90 mmol), ethylmethoxyacetate (0.11 g, 0.99 mmol) and CAL-B (60 mg) were dissolved in 2-methyltetrahydrofuran (5 mL) and stirred at 60°C for 20 h. The sample was acetylated with acetyl chloride (1.1 eq) and triethylamine (1.5 eq) in methylene chloride for one hour. The suspension was washed with hydrogen chloride (1:1, v/v) and sodium hydrogen carbonate. The solvent was removed *in vacuo.* Enantiomeric excess and conversion were determined via HPLC.

### (S)-4-cyano-1-aminoindane (2)

**Yield:** 23 mg (0.15 mmol) calculated
**¹H-NMR** (500 MHz,DMSO-d6) δ/ ppm: 1.74 (dq, 1H, ³*J*= 8.69, 12.62, C**H**₂), 2.44 (ddt, 1H, ³*J*= 15.6, 7.8, ⁴*J*= 3.2, C**H**₂), 2.88 (dt, 1H, ³*J*= 16.6, 8.4, C**H**₂), 3.04 (ddd, 1H, ³*J*= 16.5, 8.7, ⁴*J* = 3.1, C**H**₂), 4.31 (t, 1H, ³*J*= 7.6 Hz, NHC**H**), 7.40 (t, 1H, ³*J*= 7.6, Arₘ**H**), 7.64 (d, 1H, ³*J*= 7.6, Ar_{p/o}**H**), 7.72 (d, 1H, ³*J*= 7.6, Ar_{p/o}**H**).
**¹³C-NMR** (500 MHz, CDCl3) δ/ ppm: 148.8 (CH-**C**-CH₂), 147.4 (CH-**C**-CH₂), 130.9 (Ar-**C**), 128.2 (Ar-**C**), 127.5 (Ar-**C**), 117.9 (**C**≡N), 109.0 (Ar-**C**), 57.4 (N-**C**H), 37.1 (**C**H₂), 30.0 (**C**H₂).
**MS (ESI):** m/z = 159.0 [M+H]⁺, 298.1 [2M+H]⁺

### (R)-4-cyano-1-indanyl-(2-methoxyacetamid)

**Yield:** 47 mg (0.20 mmol)
**¹H-NMR** (500 MHz, CDCl3) δ/ ppm: 1.92 (dq, 1H, ³*J*= 8.60, 13.15, C**H**₂), 2.69 (dtd, 1H, ³*J*= 13.1, 8.1, ⁴*J*= 3.6, C**H**₂), 3.05 (dt, 1H, ³*J*= 16.9, 8.4, C**H**₂), 3.22 (ddd, 1H, ³*J*= 16.9, 8.9, ⁴*J* = 3.5, C**H**₂), 3.41 (s, 2H, C**H**₃), 3.96 (d, ⁴*J*= 2.4, 2H), 5.58 (q, 1H, ³*J*= 8.1 Hz, NHC**H**), 6.73 (d, 1H, ³*J*= 7.8, N**H**), 7.31 (t, 1H, ³*J*= 7.7, Arₘ**H**), 7.52 (tt, 2H, ³*J*= 7.1, ⁴*J*= 0.9 Hz, Ar_{p/o}**H**).
**¹³C-NMR** (500 MHz, CDCl3) δ/ ppm: 169.5 (**C**=O), 147.4 (CH-**C**-CH₂), 144.9 (CH-**C**-CH₂), 131.7 (Ar-**C**), 128.9 (Ar-**C**), 127.8 (Ar-**C**), 117.6 (**C≡**N), 109.3 (Ar-**C**), 72.0 (**C**H₂-O), 59.3 (**C**H₃), 53.8 (N-**C**H), 33.4 (**C**H₂), 29.9 (**C**H₂).
**MS (ESI):** m/z = 253.1 [M+Na]⁺, 483.1 [2M+Na]⁺
**IR** (neat) / cm⁻¹: 3216 (m, v, -NH), 2920 (m, v, -CH₃), 2850 (w, v, -O-CH₃), 2222 (m, v, -**C≡**N), 1654 (s, v -C=O), 1541 (w, δ, HN-C=O-), 1447 (w, δ,-CH₂), 795 (m, δ, -Ar).
**EA:** calculated for C₁₃H₁₄N₂O₂: C, 67.81; H, 6.13; N, 12.17. Found: C, 67.83; H, 6.21; N, 11.73.
**NP-HPLC:** *Daicel Chiracel* OB-H, CO₂/isopropanol 95:5, 1.5 mL/min, 20 °C, 210 nm, Rₜ₁ = 22.4 min

### Recycling Experiments with CAL-B

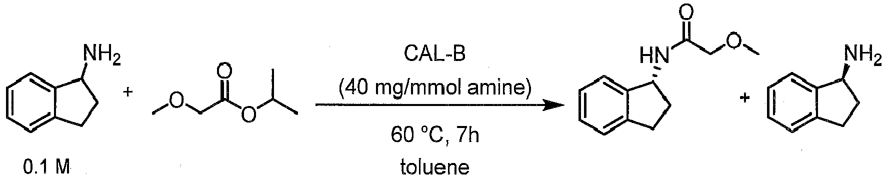

1-aminoindan (67 mg, 0.50 mmol), isopropyl methoxyacetate (73 mg, 0.55 mmol) and CAL-B (20 mg) were dissolved in 5 mL toluene and stirred at 60°C. After 7h a 200 µL sample was taken. The sample was acylated with acetyl chloride (9 mg, 0.11 mmol) and triethylamine (15 mg, 0.15 mmol). The sample was purified and analyzed. The reaction was repeated for several days.

**Table 5. Results for the Recycling Experiment with CAL-B.**

| Day | conversion/ % | *ee* (amine)/ % | *ee* (amide)/ % |
|---|---|---|---|
| 1 | 47.25 | 91.91 | 95.46 |
| 2 | 45.18 | 98.20 | 80.99 |
| 3 | 46.60 | 99.96 | 87.21 |
| 4 | 49.94 | 96.10 | 93.23 |
| 5 | 52.63 | 89.60 | 80.67 |
| 6 | 49.07 | 96.74 | 93.22 |
| 7 | 47.25 | 91.91 | 95.46 |

### Reduction of CAL-B Amount with 1-Aminoindane

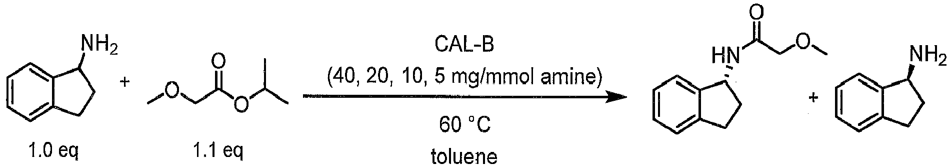

1-Aminoindane (83 mg, 0.63 mmol), isopropyl methoxyacetate (91 mg, 0.69 mmol) and CAL-B (25 mg, 13 mg, 6 mg and 3 mg) were dissolved in 1.25 mL toluene and stirred at 60°C. After 0.5 h, 1 h and 3h 100 µL samples were taken. The samples were acylated with acetyl chloride (4 mg, 0.04 mmol) and triethylamine (8 mg, 0.08 mmol). The samples were purified and analyzed.

**Table 6. Results for the Reduction of CAL-B Amount with 1-Aminoindane**

| CAL-B mg/mmol amine | t/h | conversion/% | *ee* (Amine)/% | *ee* (Amide)/% |
|---|---|---|---|---|
| 5.0 | 0.5 | 9.15 | 9.12 | 91.22 |
| 5.0 | 1 | 10.79 | 11.10 | 91.84 |
| 5.0 | 2 | 29.33 | 38.19 | 92.01 |
| 5.0 | 3 | 39.85 | 59.75 | 90.16 |
| 5.0 | 5 | 44.79 | 74.28 | 91.55 |
| 5.0 | 7 | 48.23 | 85.42 | 91.70 |
| 10.0 | 0.5 | 19.10 | 22.63 | 95.81 |
| 10.0 | 1 | 31.16 | 43.75 | 94.64 |
| 10.0 | 3 | 46.09 | 81.30 | 95.12 |
| 20.0 | 0.5 | 45.56 | 52.86 | 93.85 |
| 20.0 | 1 | 47.81 | 78.15 | 94.84 |
| 20.0 | 3 | 48.18 | 81.97 | 91.16 |
| 40.0 | 0.5 | 45.56 | 78.55 | 93.85 |
| 40.0 | 1 | 47.81 | 86.87 | 94.84 |
| 40.0 | 3 | 48.18 | 87.75 | 91.16 |

### Reduction of CAL-B Amount with 4-Cyano-1-Aminoindane

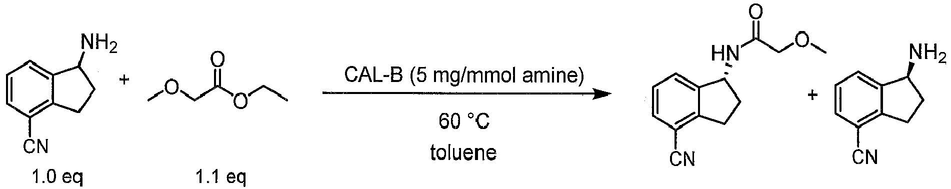

4-Cyano-1-aminoindan (37 mg, 0.23 mmol), ethyl methoxyacetate (30 mg, 0.25 mmol) and CAL-B (1.18 mg) were dissolved in 470 µL toluene and stirred at 60°C. After 0.5 h, 1 h, 3h and 5 h 50 µL samples were taken. The samples were acylated with acetyl chloride (1 eq) and triethylamine (1 eq). The samples were purified and analyzed.

**Table 7. Results for the Reduction of CAL-B Amount with 4-Cyano-1-Aminoindane**

| t/h | conversion/% | *ee* (Amine)/% | *ee* (Amide)/% |
|---|---|---|---|
| 0.5 | 33.28 | 49.20 | 97.63 |
| 1 | 44.43 | 78.95 | 96.74 |
| 3 | 54.20 | 96.67 | 82.28 |
| 5 | 56.90 | 98.88 | 74.89 |

### Multi-Step Synthesis of the Target Amine (S)-2

The substrate synthesis in this particular example has been done starting from the corresponding indanone and two step conversion into the racemic amine based on oxime formation with hydroxylamine and reduction with zinc furnishes the racemic amine *rac***-2***.* Subsequently, the lipase-catalyzed resolution with ethyl methoxyacetate as an acyl-donor was conducted at 60 °C in 2-methyltetrahydrofuran, followed by separation from the *N*-acylated product. Subsequent purification comprising the steps acidic extraction and neutralization then furnished the desired (*S*)-4-cyano-1-aminoindane, (**S**)-**2**, in an overall yield of 20% over five steps and including a resolution (with a maximum theoretical yield of 50%). The enantiomeric excess of the obtained product, (*S*)-**2**, turned out to be excellent with 99% *ee*.

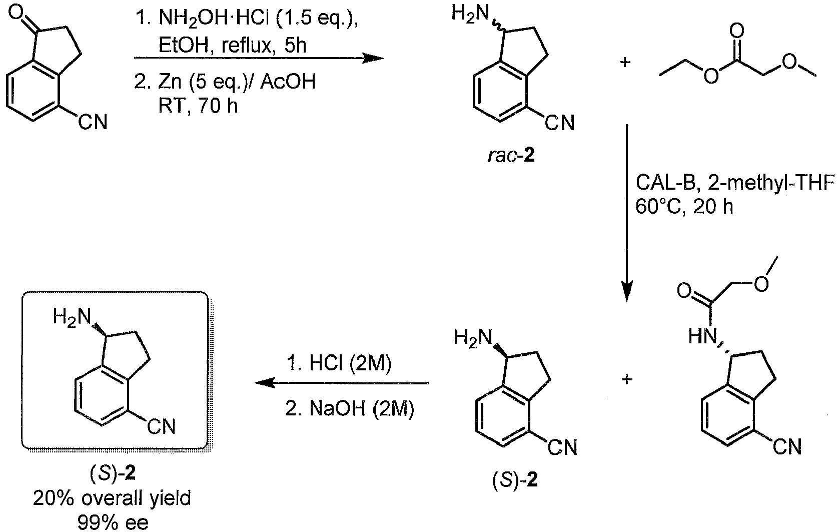

### Biotransformation at Low Biocatalyst Loading

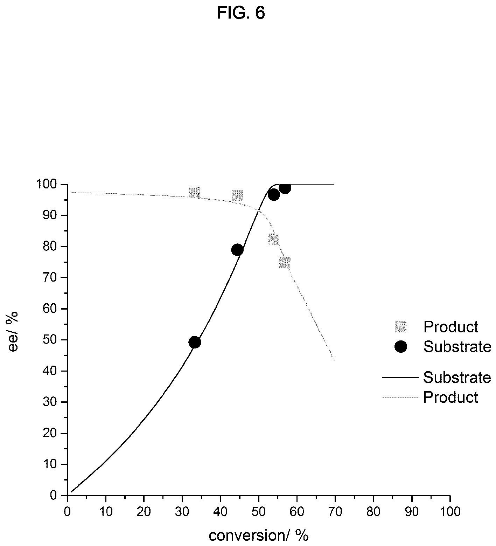

A low biocatalyst loading of 5 mg/mmol of 4-cyano-1-aminoindane, *rac*-**2**, and ethyl methoxyacetate as an acyl donor in the presence of a biocatalyst loading of 5 mg/mmol of 4-cyano-1-aminoindane, *rac*-**2**, was employed. The reaction course of this resolution is shown in Figure 6 and indicates that a high enantiomeric excess of >98% *ee* was reached at a conversion of about 57%.

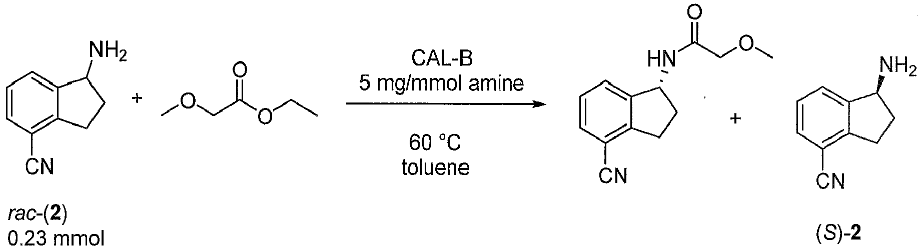

**Figure 6****.** Resolution of 4-Cyano-1-Aminoindane at Low Biocatalyst Loading of Lipase CAL-B

### Experimental Protocols and Data Related to the Development of a Novel Total Synthesis of Ozanimod

### Reduction of Naphthalene and Re-arrangement Towards 1,2-Dihydronaphthalene

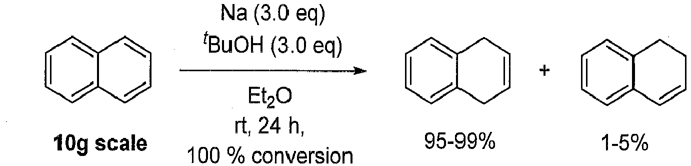

Naphthalene (10.00 g, 78 mmol, 1.00 eq) is dissolved in Et₂O (150 mL). Small pieces of sodium (5.00 g, 217 mmol, 2.78 eq) are given to the solution. The atmosphere over the sodium was nitrogen flushed. *Tert*.-BuOH (14.5 g, 196 mmol, 2.50 eq) is dissolved in Et₂O (50 mL) and dropped into the sodium suspension. After stirring 16 h at room temperature the reaction mixture was quenched with water and extracted with ethyl acetate (3 times). The combined organic layers are dried with Na₂SO₄ and separated from the solvent *in vacuo.* Conversion is calculated by NMR spectrum. Yield: 86 %.

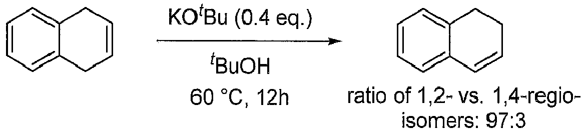

1,4-dihydronaphthalene (7.92 g, 60.1 mmol, 1 eq) is dissolved in *tert*.-butanol (6 mL). Potassium *tert*.-butylate (2.5 g, 22.3 mmol, 0.4 eq) is given to the solution and the mixture is stirred for 12 h at 60°C under argon atmosphere. The reaction mixture is admixed with DCM (10 mL) and washed three times with water (15 mL). The solvent was removed *in vacuo* and the product recovered as a slightly yellowish liquid. The reaction control was carried out by GC and the purity of the product was determined by 1H NMR, leading to a ratio of the two region-isomers of 97:3 after 12h reaction time with a preference for the desired 1,2-dihydronaphthalene.
¹H-NMR (500 MHz, CDCl₃): *δ* (ppm) =2.28 (m, 2 H, H-9), 2.75 (t, 2 H, H-10), 5.99 (m, 1 H, H-8), 6.42 (m, 1 H, H-7), 6.85-7.29 (m, 4 H, H-1, -2, -3, -6). ¹³C-NMR (500 MHz, CDCl₃): *δ* (ppm) =23.2 (C-10), 27.5 (C-9), 125.9 (CH), 126.4 (CH) 126.8 (CH), 127.5 (CH), 127.8 (CH), 128.5 (CH), 134.2 (C), 135.4 (C).

### Oxidative Cleavage of the C=C-Double Bond in 1,2-Dihydronaphthalene

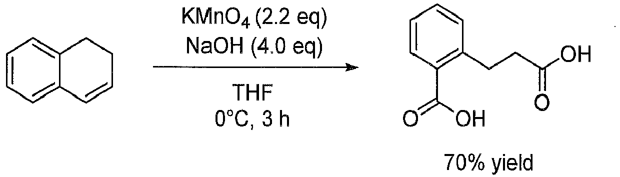

1,2-Dihydronapthalene (15.00 g, 115 mmol, 1.00 eq) was dissolved in methylene chloride (50 mL) is then slowly added drop wise to a potassium permanganate solution (2000 mL, 230 mmol, 230 mM, 4.00 eq). The reaction mixture was stirred for 3h at 0°C. After adding sodium hydroxide (32 g, 800 mmol, 6.96 eq) the solution was filtrated to separate manganese dioxide.

The filtrate is extracted by methylene chloride (3 times 1000 mL). The aqueous layer was acidified with concentrated hydrochloric acid (70 mL, 840 mmol, 12 M, 7.30 eq) to pH 12 to yield a precipitate. After filtration the diacid can be isolated with a yield of 70% (15.53 g, 80 mmol).
m/z (M+Na): 217.0; m/z (M-H): 192.8.
¹H-NMR (500 MHz, CDCl₃): *δ* (ppm) =2.73 (t, 2 H, H-10), 3.42 (t, 2 H, H-11), 7.35 (m, 2 H, H^{ar}), 7.52 (m, 1 H, H^{ar}), 8.06 (d, ³*J*_{HH}=7.7 Hz, 1 H, H^{ar}). ¹³C-NMR (500 MHz, DMSO): *δ* (ppm) =30.0 (C-10), 36.3 (C-11), 127.2 (CH), 131.2 (CH) 131.4 (CH), 131.7 (CH), 132.7 (CH), 142.8 (CH), 167.6 (C-7), 174.7 (C-12).

### Intramolecular Friedel-Crafts-Acylation under Indan-1-one Formation

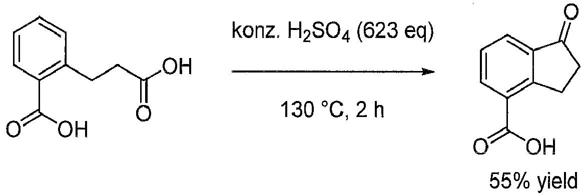

3-(2-Carboxyphenyl)propionic acid (500 mg, 2.57 mmol, 1.00 eq) was dissolved in sulfuric acid (30 mL). The reaction mixture was stirred for 2h at 130°C. The complete reaction mixture was given on ice (20 g). The solution was stored at 8°C for 65 h to yield a precipitate. After filtration 55 % of the clean product (analytic by NMR) could be isolated. The color of the product should be colorless but the isolated product was brown. The brown substance could be dissolved with Ethanol. A re-crystallization was also possible.
m/z (M-H): 174.8.
¹H-NMR (500 MHz, CDCl₃): *δ* (ppm) =3.16-3.22 (m, 2 H, H-7), 3.54-3.58 (m, 2 H, H-8), 7.62 (m, 1 H, H-1), 8.03 (d, ³*J*_{HH}=7.6 Hz, 1 H, H-2), 8.38 (d, ³*J*_{HH}=8.0 Hz, 1 H, H-6). ¹³C-NMR (500 MHz, DMSO): *δ* (ppm) =27.4 (C-7), 36.2 (C-8), 127.6 (C-1), 128.2 (C-1) 129.5 (C-3), (C-6), 136.6 (C-2), 138.4 (C-5), 156.8 (C-4), 167.3 (C-11), 206.4 (C-9).

### Derivatization of the 4-Carboxy Substituent of Indanone

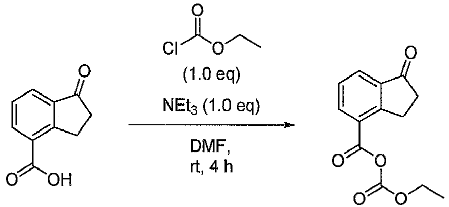

4-Carboxy-1-indanon (580 mg, 4.10mmol, 1.00 eq) and triethylamine (600 µL, 4.30 mmol, 1.0 eq) were dissolved in DMF (11 mL) and cooled to 0°C. Ethylchloroformate (420 µL, 4.10 mmol, 1.00 eq). The reaction mixture was stirred for 2h at rt. After quenching with water (3 mL) the mixture is extracted with DCM (3x 7 mL). The combined organic layers are washed with water (15 mL) and dried with MgSO₄. The solvent was removed to yield an brown oil (60 %).
m/z (M+Na): 271.1; m/z (accurate mass): 271.0577.
¹H-NMR (500 MHz, CDCl₃): *δ* (ppm) =1.45 (t, 3 H, H-18), 2.77 (m, 2 H, H-7), 3.55 (m, 2 H, H-8), 4.45 (q, 2 H, H-17), 7.56 (m, 1 H, H-1), 8.05 (d, ³*J*_{HH}=7.6 Hz, 1 H, H-2), 8.32 (d, ³*J*_{HH}=8.0 Hz, 1 H, H-6). ¹³C-NMR (500 MHz, CDCl₃): *δ* (ppm) =13.9 (C-18), 27.1 (C-7), 36.0 (C-8), 66.0 (C-17), 125.8 (C-1), 127.9 (C-3), 129.6 (C-6), 137.0 (C-2), 138.8 (C-5), 148.5 (C-4), 148.9 (C-11), 158.0 (C-14), 205.8 (C-9).

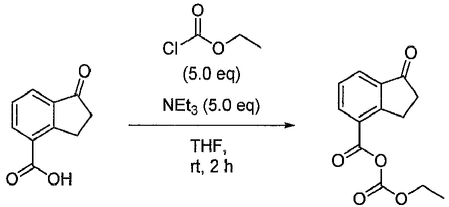

4-Carboxy-1-indanon (200 mg, 1.14mmol, 1.00 eq) and triethylamine (551 µL, 5.79 mmol, 5.0 eq) were dissolved in THF (5 mL) and cooled to 0°C.

Ethylchloroformate (540 µL, 5.67 mmol, 5.00 eq). The reaction mixture was stirred for 2 h at rt. After quenching with water (3 mL) the mixture is extracted with DCM (3x 7 mL). The combined organic layers are washed with water (15 mL) and dried with MgSO₄. The solvent was removed to yield an brown oil (85 %).
m/z (M+Na): 271.1; m/z (accurate mass): 271.0577.
¹H-NMR (500 MHz, CDCl₃): *δ* (ppm) =1.45 (t, 3 H, H-18), 2.77 (m, 2 H, H-7), 3.55 (m, 2 H, H-8), 4.45 (q, 2 H, H-17), 7.56 (m, 1 H, H-1), 8.05 (d, ³*J*_{HH}=7.6 Hz, 1 H, H-2), 8.32 (d, ³*J*_{HH}=8.0 Hz, 1 H, H-6). ¹³C-NMR (500 MHz, CDCl₃): *δ* (ppm) =13.9 (C-18), 27.1 (C-7), 36.0 (C-8), 66.0 (C-17), 125.8 (C-1), 127.9 (C-3), 129.6 (C-6), 137.0 (C-2), 138.8 (C-5), 148.5 (C-4), 148.9 (C-11), 158.0 (C-14), 205.8 (C-9).

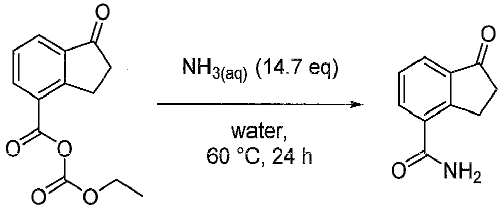

The mixed anhydride (100 mg, 0.40 mmol, 1.00 eq) was given to an ammonia solution (2.5 mL, 25 %, 14.7 eq) and heated to 60°C. The reaction mixture was stirred for 24 h at rt. The mixture was extracted with DCM (3x 5 mL). The combined organic layers are washed with water (15 mL) and dried with MgSO₄. The solvent was removed to yield a brown oil (70 %).
m/z (M-H): 173.8.
¹H-NMR (500 MHz, CDCl₃): *δ* (ppm) =1.45 (t, 3 H, H-18), 2.77 (m, 2 H, H-7), 3.55 (m, 2 H, H-8), 4.45 (q, 2 H, H-17), 7.56 (m, 1 H, H-1), 8.05 (d, ³*J*_{HH}=7.6 Hz, 1 H, H-2), 8.38 (d, ³*J*_{HH}=8.0 Hz, 1 H, H-6). ¹³C-NMR (500 MHz, CDCl₃): *δ* (ppm) =26.3 (C-7), 36.2 (C-8), 126.8 (C-1), 127.7 (H-6), 132.0 (C-2), 132.8 (C-3), 138.5 (C-5), 154.7 (C-4), 168.7 (C-11), 206.4 (C-9).

The resulting primary amide (150 mg, 0.85 mmol, 1.00 eq) and triethylamine (356 µL, 2.55 mmol, 3.00 eq) were suspended in toluene (5 mL). Phosphorus pentoxide (48 mg, 0.34 mmol, 0.40 eq) was added and the mixture was heated to 80 C. The reaction mixture was stirred for 5h. After quenching with water (3 mL) the mixture is extracted with DCM (3x 7 mL). The combined organic layers are washed with water (15 mL) and dried with MgSO₄. The solvent was removed to yield a brown oil (29 %) of the target compound (OZA-5).
m/z (M-Na): 179.0.
¹H-NMR (500 MHz, CDCl₃): *δ* (ppm) =2.82 (m, 2 H, H-7), 3.36 (m, 2 H, H-8), 7.54 (t, 1 H, H-1), 7.91 (d, ³*J*_{HH}=7.6 Hz, 1 H, H-6), 7.99 (d, ³*J*_{HH}=8.0 Hz, 1 H, H-2). ¹³C-NMR (500 MHz, CDCl₃): *δ* (ppm) =25.3 (C-7), 35.8 (C-8), 111.5 (C-3), 116.1 (C-11), 128.1 (C-6), 128.3 (C-1), 137.7 (C-2), 138.2 (C-5), 157.7 (C-4), 204.5 (C-9).

### Reductive Amination of 4-Cyanoindanone in a Two-Step Process

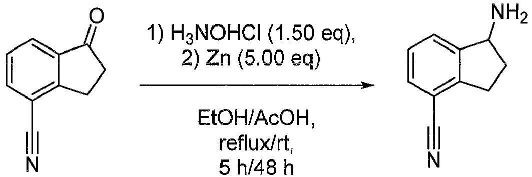

The ketone (500 mg, 3.18 mmol, 1.00 eq) was dissolved in EtOH (10 mL). Hydroxyammonium chloride (3.32 g, 47.72 mmol, 1.50 eq) was added and the mixture was heated to reflux for 2h. The solvent is evaporated under reduced pressure and the intermediate is dissolved in acetic acid (10 mL). To the solution is added Zn (10.40 g, 159.1 mmol, 5.0 eq). The suspension is stirred under Ar atmosphere for 48 h. The crude product was extracted with methylene chloride and hydrochloric acid (1M) (3 times). The pH of the aqueous phase was changed to 14 and the phase was extracted with methylene chloride (3 times). The product (2.16g, 1.38 mmol, 42%) was isolated after evaporation of the solvent.

The synthesis of the desired racemic 4-cyano-1-aminoindane leads to a yield of 42%.
m/z (M+H): 159.0.
¹H-NMR (500 MHz, CDCl₃): *δ* (ppm) =1.70 (m, 1 H, H-7), 2.53 (m, 1 H, H-7), 2.89 (m, 2 H, H-8), 3.10 (m, 2 H, H-8), 4.35 (t, ³*J*_{HH}=7.6 Hz, 1 H, H-9), 7.24 (t, ³*J*_{HH}=7.6 Hz, 1 H, H-1), 7.42 (d, ³*J*_{HH}=7.7 Hz, 1 H, H-2), 7.49 (d, ³*J*_{HH}=7.7 Hz, 1 H, H-2). ¹³C-NMR (500 MHz, CDCl₃): *δ* (ppm) =29.7 (C-7), 37.1 (C-8), 57.3 (C-9), 108.9 (C-3), 117.8 (C-11), 127.4 (C-1), 128.1 (C-2), 130.8 (C-6), 147.3 (C-4), 148.8 (C-5).

### Amidoxime Formation (Model Reaction)

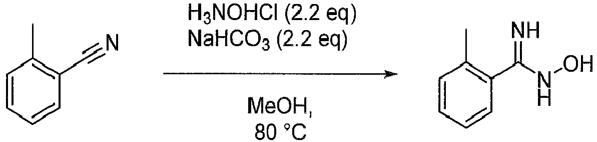

Hydroxylammonium chloride (1.399 g, 20 mmol, 2.00 eq) was dried in vacuo and given to MeOH (10 mL), NaHCO₃ (1815 mg, 2.16 mmol, 2.16 eq) and *ortho*-Tolunitril (1170 µL, 10 mmol, 1.00 eq). After 7.3 h at 80 °C, the reaction is quenched with water (5 mL) and extracted with CHCl₃ (3x 10 mL). The combined organic layers are washed with water (2x 15 mL) and dried with MgSO₄. The solvent was removed to yield the following product ratio (analyzed by 1H NMR): 9% nitrile, 72% amidoxime, 19% amide.
¹H-NMR (500 MHz, CDCl₃): *δ* (ppm) =2.39 (s, 3 H, H-7), 5.45 (s, 1 H, H-10) 7.14-7.63 (m, 4 H, H^{ar}). ¹H-NMR (500 MHz, DMSO-d₆): *δ* (ppm) =2.39 (s, 3 H, H-7), 5.74 (s, 2 H, H-9, H-10) 7.19-7.32 (m, 4 H, H^{ar}), 9.38 (s, 1 H, H-11). ¹³C-NMR (500 MHz, DMSO-d₆): *δ* (ppm) =19.8, 125.4, 128.6, 128.9, 130.2, 134.3, 136.4, 152.5.

### Formation of the Oxadiazole Heterocycle (Model Reaction)

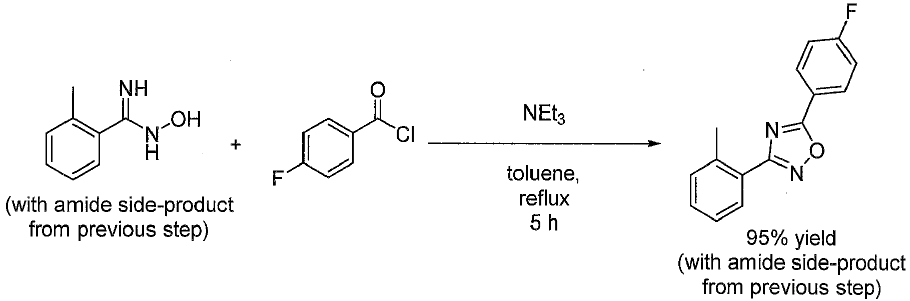

A mixture of amidoxime (105 mg, 0.7 mmol, 1.00 eq) and amide (resulting of the previous step, 95 mg, 72 mmol, 1.03 eq) was dissolved in THF (10 mL) and pyridine (2 mL). Para-fluorobenzoic acid chloride (100 µL, 0.83 mmol, 1.19 eq) is given to the solution. After 66 h at reflux, the reaction is quenched with water (10 mL) and extracted with CHCl₃ (3x 20 mL). The combined organic layers are washed with water (2x 15 mL) and dried with MgSO₄. The solvent was removed to yield a light yellow solid (analyzed by 1H NMR). Purification was done by column chromatography (1. Snap Ultra 50 mg®; eluent: CyHex/EtOAc 90:10 - 30:70, 2. length: 16 cm; ø: 4 cm; eluent CyHex/EtOAc 35:65). After purification a colorless solid (119 mg, 0.42 mmol, 95 %) was isolated.
¹H-NMR (500 MHz, CDCl₃): *δ* (ppm) =2.68 (s, 3 H, H-23), 7.24 (m, 2 H, H-4 & H-6), 7.35 (m, 2 H, H-19 & H-21), 7.41 (m, 1 H, H-20), 8.07 (m, 1 H, H-22), 8.24 (m, 2 H, H-1 & H-3). ¹³C-NMR (500 MHz, CDCl₃): *δ* (ppm) =22.3, 116.62, 120.9, 126.2, 130.2, 130.7, 13.8, 131.5, 138.4, 165.6, 169.8, 173.9. ¹⁹F-NMR (500 MHz, CDCl₃): *δ* (ppm) = -105.3

## Claims

1. A process for the enantioselective biocatalytic preparation of 4-substituted 1-aminoindanes of general formula 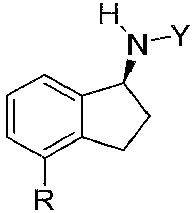 wherein
R is selected from the group consisting of halogen; -CN; -C(=O)H; -C(=N-OH)H; -C(=O)NH₂; -C(=O)OH; -C(=NH)NH-OH; -C(=N-OH)NH₂; and -COOR¹, wherein R¹ is selected from the group consisting of H; C₁ to Cs-alkyl; arylalkyl, wherein alkyl is C₁ to Cs-alkyl; -CH₂-Hal, wherein Hal is F, Cl or Br; -CH₂-O-alkyl, wherein alkyl is C₁ to Cs-alkyl; optionally substituted aryl; and optionally substituted heteroaryl;
Y is selected from the group consisting of H; -C(=O)R^{1y}; and - C(=O)OR^{1y}, wherein R^{1y} is selected from the group consisting of H; C₁ to C₈-alkyl; arylalkyl, wherein alkyl is C₁ to C₈-alkyl; -CH₂-Hal, wherein Hal is F, Cl or Br; -CH₂-O-alkyl, wherein alkyl is C₁ to Cs-alkyl; optionally substituted aryl; and optionally substituted heteroaryl;
via a route selected from the group consisting of
A resolution of a racemic or enantiomerically-enriched amine mixture of 4-substituted indan-1-amine compounds of general formulae (*S*)-II and (*R*)-II 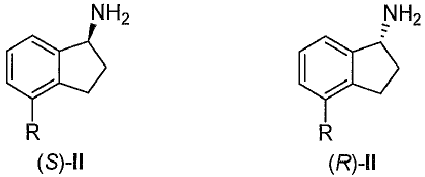
wherein R is as defined above
via an enantioselective acylation in the presence of a biocatalyst;
B resolution of a racemic or enantiomerically-enriched amide mixture of N-acylated 4-substituted indan-1-amine compounds of general formulae (*S*)-III and (*R*)-III 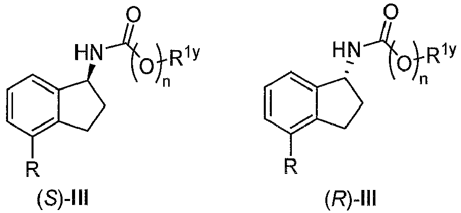
wherein R and R^{1y} are as defined above and n is 0 or 1,
via an enantioselective hydrolysis in the presence of a biocatalyst;
C asymmetric reductive amination of a 4-substituted 1-indanone with a transaminase, wherein the substituent at position 4 is R as defined above; or
D asymmetric reductive amination of a 4-substituted 1-indanone with an amine dehydrogenase, wherein the substituent at position 4 is R as defined above.

2. The process according to claim 1, **characterized in that** the route is route A or route B, preferably route A.

3. The process according to claim 2, wherein the biocatalyst is selected from the group of hydrolases, preferably wherein the hydrolases are selected from the group consisting of lipases, esterases and proteases, most preferably wherein the hydrolase is lipase.

4. The process according to claim 2 or 3, **characterized in that** in route A a racemic amine mixture or an enantiomerically-enriched amine mixture of 4-substituted indan-1-amine compounds of general formulae (S)-II and (*R*)-II is added together with an acyl donor to a biocatalyst, preferably wherein the biocatalyst is a lipase.

5. The process according to claim 4, **characterized in that** in route A a racemic amine mixture or an enantiomerically-enriched amine mixture of 4-substituted indan-1-amine compounds of general formulae (S)-II and (*R*)-II is added together with an acyl donor to a biocatalyst, preferably wherein the biocatalyst is a lipase, according to the following reaction Scheme 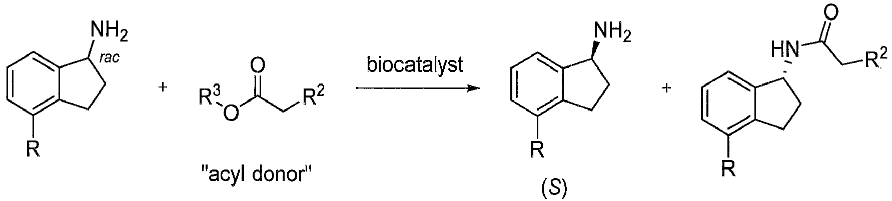 wherein
R is as defined in claim 1, preferably wherein R is -CN, -COOMe, or -Br; R² is selected from the group consisting of a C₁ to C₂₀-alkyl group; a C₁ to C₂₀-alkoxy group; an optionally substituted C₆ to C₂₀-aryl group; an optionally substituted 5- or 6-membered heteroaryl group; halogen and an acyl group of formula -COOR⁴, wherein R⁴ is selected from H, and C₁ to Cs-alkyl, preferably -CH₃ or -C₂H₅; preferably wherein R² is methoxy or -COOC₂H₅ or -CI;
R³ is selected from a C₁ to C₈- or C₁ to C₆-alkyl group, preferably wherein R³ is a C₁ to C₄-alkyl group, more preferably wherein R³ is methyl, ethyl or isopropyl; -CH₂-Hal wherein Hal represents -F, -Cl or - Br; -CH₂-O-alkyl wherein alkyl represents C₁ to Cs-alkyl; an optionally substituted aryl group; and an optionally substituted 5- or 6-membered heteroaryl group;
wherein the reaction is conducted in an organic medium in the presence of an organic solvent or under solvent-free conditions.

6. The process according to any one of claims 1 to 5, **characterized in that** R is selected from the group consisting of -Br, -CN and -COOCH₃, preferably wherein R is -CN or -Br, most preferably wherein R is -CN.

7. The process according to any one of claims 2 to 6, **characterized in that** it is carried out in the absence of any additional solvent or in the presence of an additional organic solvent, preferably wherein the organic solvent is at least one selected from the group consisting of n-heptane, 2-methoxy-2-methylpropane, methyl-tert.-butylether, 2-methyl-tetrahydrofuran, methylcyclohexane, toluene and any combination of any thereof, preferably wherein the organic solvent is n-heptane and/or methyl-tert.-butylether.

8. The process according to any one of claims 2 to 7, **characterized in that** the lipase is selected from the group consisting of lipase B from *Candida antarctica* (CAL-B), lipase from *Candida rugosa* (CR lipase), lipase from *Burkholderia cepacia* (BC lipase) and lipase PS from *Burkholderia cepacia,* preferably wherein the lipase is CAL-B.

9. The process according to any one of claims 2 to 8, **characterized in that** the acyl donor is selected from the group consisting of ethyl methoxyacetate, diethyl malonate, and isopropyl methoxyacetate, preferably wherein the acyl donor is isopropyl methoxyacetate.

10. The process according to any one of claims 2 to 9, **characterized in that** the reaction is conducted under one of the following conditions a), b) or d), or wherein the reaction is conducted under a condition selected from conditions a) and b); a) and c); b) and c); and a), b) and c):
a) for a period of time sufficient to reach 99 % *ee* of the desired 4-substituted 1-aminoindanes, preferably 30 minutes to 48 hours, more preferably for at least 5 h;
b) at a temperature range of 10 to 90 °C, preferably at 40 to 60 °C;
c) at a substrate: enzyme ratio of 1 to 50 mg, preferably 5 mg, CAL-B per mmol substrate;
preferably in solution in the presence of n-heptane as the solvent.

11. The process according to any one of claims 2 to 10, **characterized in that** the racemic mixture or the enantiomerically-enriched amine mixture of 4-substituted indan-1-amine compounds of general formulae (*S*)-II and (*R*)-II is obtained from or is obtainable from the corresponding 4-substituted 1-indanone in which the substituent R at the 4-position is as defined in claim 1, preferably by
i) the formation of a ketoxime, particularly by condensation of the 4-substituted 1-indanone with hydroxylamine, followed by
ii) the reduction of the ketoxime, preferably in the presence of zinc, to the racemic amine mixture.

12. The process according to any one of claims 2 to 11, **characterized in that** the undesired enantiomer(s) resulting from the enzymatic acylation are recovered and recycled/reused.

13. The process according to any one of claims 2 to 12, **characterized in that** a racemic or enantiomerically-enriched amine mixture of 4-substituted indan-1-amine compound of general formulae (*S*)-II and (*R*)-II, wherein R is -CN and Y is hydrogen, is acylated with isopropyl methoxyacetate in the presence of CAL-B in n-heptane and/or methyl-tert.-butylether as the solvent.

14. The process according to any one of claims 1 to 13, **characterized in that** the initial 4-substituted indane-1-one compound is obtainable from or obtained from naphthalene.

15. A process for the preparation of Ozanimod, said process comprising or consisting of the steps, in the given order, of
I providing naphthalene,
II reducing naphthalene to 1,2- and/or 1,4-dihydronaphthalene,
IIa optionally rearranging 1,4-dihydronaphthalene to 1,2-dihydronaphthalene,
III cleaving the ethylenic C=C-double bond in 1,2-dihydronaphthalene to provide a dicarboxylic acid,
IV cyclization of the product to 4-carboxy-1-indanone,
V derivatization of the 4-carboxy-1-indanone to 4-cyano-1-indanone,
VI converting the 4-cyano-1-indanone to 4-cyano-1-aminoindane in the S-configuration, either directly or via the racemate,
VII protecting the amino-group of the 4-cyano-1-aminoindane with a protecting group,
VIII converting the nitrile-group to an amidoxime group,
IX converting the amidoxime-group to a 1,4-oxadiazole heterocyclic-group via condensation with 3-cyano-4-isopropoxy benzoic acid or 3-cyano-4-isopropoxy benzoic acid derivative,
X substituting the protecting group on the amino group with a hydroxyethyl group to yield Ozanimod,
wherein the conversion of the 4-cyano-1-indanone to 4-cyano-1-aminoindane in the S-configuration in step VI is carried out by process according to any one of claims 1 or 2 to 14.

16. A process for the preparation of Ozanimod, said process comprising or consisting of the steps, in the given order, of
VI providing 4-cyano-1-aminoindane in the S-configuration, obtained by the process according to any one of claims 1 or 2 to 14,
VII protecting the amino-group of the 4-cyano-1-aminoindane with a protecting group,
VIII converting the nitrile-group to an amidoxime group,
IX converting the amidoxime-group to a 1,4-oxadiazole heterocyclic-group via condensation with 3-cyano-4-isopropoxy benzoic acid or 3-cyano-4-isopropoxy benzoic acid derivative,
X substituting the protecting group on the amino group with a hydroxyethyl group to yield Ozanimod.
